# FASCICULE DE BREVET EUROPEEN

(11) **EP 1 893 228 B1**
(45) Date de publication et mention de la délivrance du brevet: **23.03.2016**
(21) Numéro de dépôt: 06778626.9
(22) Date de dépôt: 20.06.2006
(51) Int. Cl.: A61K 38/16, C07K 14/18, C12N 15/09, C12N 15/62, C12N 15/86, C07K 16/10

(54) **POLYPEPTIDES CHIMERIQUES ET LEURS APPLICATIONS THÉRAPEUTIQUES CONTRE UNE INFECTION À FLAVIVIRIDAE**
CHIMÄRISCHE POLYPEPTIDE UND IHRE THERAPEUTISCHE VERWENDUNG GEGEN FLAVIVIRIDAE-INFEKTIONEN
CHIMERIC POLYPEPTIDES AND THE THERAPEUTIC USE THEREOF AGAINST A FLAVIVIRIDAE INFECTION

(30) Priorité: 20.06.2005 CA 2508266
(43) Date de publication de la demande: 05.03.2008
(73) Titulaire: INSTITUT PASTEUR, 75724 Paris Cedex 15 (FR); Centre National de la Recherche Scientifique, 75794 Paris Cedex 16 (FR)
(72) Inventeur: TANGY, Frédéric, 93260 Les Lilas (FR); LUCAS-HOURANI, Marianne, 75017 Paris (FR); NAVARRO-SANCHEZ, Erika, 93100 Montreuil-sous-Bois (FR); FRENKIEL, Marie-Pascale, 92300 Levallois-Perret (FR); BEDOUELLE, Hugues, 75015 Paris (FR); COMBREDET, Chantal, 75017 Paris (FR); DESPRES, Philippe, 92250 La Garenne-Colombes (FR)
(74) Mandataire: Desaix, Anne
(86) Numéro de dépôt international: PCT/FR2006/001396
(87) Numéro de publication internationale: WO 2006/136697

(56) Documents cités:
- WO-A-2005/111221
- WO-A2-01/96376
- WO-A2-2004/076619
- CATTEAU ADELINE ET AL: "Dengue virus M protein contains a proapoptotic sequence referred to as ApoptoM." JOURNAL OF GENERAL VIROLOGY, vol. 84, no. 10, octobre 2003 (2003-10), pages 2781-2793, XP002405613 ISSN: 0022-1317
- CATTEAU ADELINE ET AL: "Expression of dengue ApoptoM sequence results in disruption of mitochondrial potential and caspase activation." BIOCHIMIE (PARIS), vol. 85, no. 8, août 2003 (2003-08), pages 789-793, XP002405614 ISSN: 0300-9084
- COLOMBAGE G ET AL: "DNA-Based and Alphavirus-Vectored Immunisation with PrM and E Proteins Elicits Long-Lived and Protective Immunity against the Flavivirus, Murray Valley Encephalitis Virus" VIROLOGY, ACADEMIC PRESS,ORLANDO, US, vol. 250, no. 1, 10 octobre 1998 (1998-10-10), pages 151-163, XP004445627 ISSN: 0042-6822
- FONSECA B A L ET AL: "RECOMBINANT VACCINIA VIRUSES CO-EXPRESSING DENGUE-1 GLYCOPROTEINS PRM AND E INDUCE NEUTRALIZING ANTIBODIES IN MICE" VACCINE, BUTTERWORTH SCIENTIFIC. GUILDFORD, GB, vol. 12, no. 3, 1994, pages 279-285, XP009018695 ISSN: 0264-410X
- CRILL W D ET AL: "Monoclonal antibodies that bind to domain III of dengue virus E glycoprotein arethe most efficient blockers of virus adsorption to Vero cells" JOURNAL OF VIROLOGY, THE AMERICAN SOCIETY FOR MICROBIOLOGY, US, vol. 75, no. 16, août 2001 (2001-08), pages 7769-7773, XP002982315 ISSN: 0022-538X
- DESPRÈS P ET AL: "Live measles vaccine expressing the secreted form of the west nile virus envelope glycoprotein protects against west nile virus encephalitis" JOURNAL OF INFECTIOUS DISEASES 15 JAN 2005 UNITED STATES, vol. 191, no. 2, 15 janvier 2005 (2005-01-15), pages 207-214, XP009074536 ISSN: 0022-1899
- MOTA J ET AL: "Induction of protective antibodies against dengue virus by tetravalent DNA immunization of mice with domain III of the envelope protein" VACCINE, BUTTERWORTH SCIENTIFIC. GUILDFORD, GB, vol. 23, no. 26, 16 mai 2005 (2005-05-16), pages 3469-3476, XP004852137 ISSN: 0264-410X
- WANG SONGLI ET AL: "PrM- and cell-binding domains of the dengue virus E protein" JOURNAL OF VIROLOGY, vol. 73, no. 3, mars 1999 (1999-03), pages 2547-2551, XP002405616 ISSN: 0022-538X
- HERMIDA ET AL: "A recombinant fusion protein containing the domain III of the dengue-2 envelope protein is immunogenic and protective in nonhuman primates" VACCINE, BUTTERWORTH SCIENTIFIC. GUILDFORD, GB, vol. 24, no. 16, 12 avril 2006 (2006-04-12), pages 3165-3171, XP005344486 ISSN: 0264-410X

## Description

### DOMAINE DE L'INVENTION

La présente invention concerne la construction d'un polypeptide chimérique utile dans la prévention ou dans le traitement d'une infection à *Flaviviridae.* La présente invention porte également sur l'utilisation du polypeptide chimérique dans l'élaboration de vecteurs viraux recombinants, tel un vecteur viral vivant rougeole pour agir en tant que vaccin.

### BRÈVE DESCRIPTION DE L'ART ANTÉRIEUR

Les flavivirus (famille des *Flaviviridae*) sont des virus enveloppés dont le génome est une molécule d'ARN de polarité positive. Le flavivirion est composé de trois protéines structurales désignées C (protéine du core), M (protéine de membrane) et E (protéine d'enveloppe). La protéine E qui est exposée à la surface du virus est responsable des fonctions biologiques principales du virion incluant l'attachement du virus et la fusion membranaire spécifique. La traduction de l'ARN génomique produit une polyprotéine précurseur qui est clivée simultanément par des protéases cellulaires et virales pour générer les protéines virales individuelles : trois protéines structurales C, prM (le précurseur glycosylé de la protéine M), E, et sept protéines non-structurales NS1 à NS5. La réplication des flavivirus s'opère dans le cytoplasme des cellules infectées. Les flavivirus ont des cycles de transmission naturels complexes qui impliquent plusieurs hôtes naturels (en majorité des mammifères et des oiseaux) et des vecteurs, ces derniers étant des arthropodes hématophages tels que des tiques et des moustiques. Ces virus sont la cause principale de maladies humaines sévères telles que des manifestations hémorragiques ou des syndromes méningo-encéphalitiques. Le virus de la dengue (DEN) est un des deux flavivirus majeurs reconnus comme étant la cause de maladies hémorragiques sévères à travers le monde.

La dengue est une maladie émergeante et une préoccupation de santé publique. Depuis plus de 50 ans, le développement de candidats vaccins vivants-atténués contre les quatre sérotypes du virus de la dengue s'est poursuivi. La recherche d'un vaccin se bute à l'absence d'un modèle animal mimant la maladie causée par le virus de la dengue, soit la fièvre hémorragique de la dengue et le syndrome de choc qui lui est associé. L'efficacité des vaccins peut être évaluée chez la souris en utilisant une souche neurovirulente de virus DEN adaptée à la souris, qui tue les animaux adultes après une inoculation intracérébrale. Cependant, les études fondées sur ce type de modèles murins ne reflètent pas l'activité des virus de la dengue chez l'homme. Chez le singe, la protection ne peut être démontrée qu'en mesurant la réduction de virémie après une épreuve infectieuse expérimentale. Ainsi, le test ultime pour un vaccin contre la dengue doit être fondé sur des essais cliniques réalisés chez l'homme. La plupart des candidats vaccins atténués qui ont été développés ces dernières 50 années ont été soit trop réactogéniques soit insuffisamment immunogènes dans les essais cliniques. Plusieurs candidats tétravalents atténués sont aujourd'hui en essai clinique de phase I ou II. La difficulté dans le développement de ce type de vaccins vivants-atténués tétravalents semble être d'obtenir un mélange équilibré des quatre valences afin de générer une immunité protectrice contre les quatre sérotypes.

Différents candidats vaccins chimériques vivants sont également en cours de développement. Ils sont basés sur l'échange de gènes structuraux homologues entre différents flavivirus. Plusieurs types de virus chimériques intertypiques ont été construits et testés chez la souris ou le singe. Des virus chimériques ont été construits avec la technologie Chimerivax® basée sur le virus vaccinal de la fièvre jaune (YF-17D). Des mélanges tétravalents de ces chimères YF-17D/DEN ont été testés chez le singe rhésus. Là encore, l'équilibre entre les quatre virus chimériques est difficile à obtenir pour une immunisation uniforme. Un essai clinique de phase I/II est en préparation actuellement pour tester un vaccin Chimerivax-dengue 2. D'autres approches incluent des candidats vaccins sous-unitaires produits à partir d'un vecteur d'expression, et des candidats de type ADN nu qui sont en développement pré-clinique. Ainsi, le développement d'un vaccin tétravalent contre le virus de la dengue demeure un problème non résolu, un des plus importants sur la liste de l'Organisation Mondiale de la Santé.

Il existe donc le besoin de développer de nouveaux candidats vaccins efficaces, faciles à produire et à formuler pour la prévention ou le traitement d'une infection par un virus de la famille des *Flaviviridae* comme le virus de la Dengue.

### RÉSUMÉ DE L'INVENTION

La présente invention concerne un polypeptide chimérique et ses applications dans la prévention ou dans le traitement d'une infection à *Flaviviridae.*

Plus particulièrement, la présente invention a pour objet un polypeptide chimérique comprenant un peptide de sous-domaine de la protéine E de *Flaviviridae* lié à un peptide de sous-domaine de la protéine de membrane M de *Flaviviridae* et, le cas échéant, un segment de liaison liant le peptide de sous-domaine de la protéine E au peptide de sous-domaine de la protéine M, dans lequel le peptide de sous-domaine de la protéine M consiste en:
- l'ectodomaine 1-40 comprenant une séquence en acides aminés allant de la position 123 à 162 de la SEQ ID NO : 3; ou
- la séquence apoptoM comprenant une séquence en acides aminés allant de la position 154 à 162 de la SEQ ID NO : 3 ou allant de la position 122 à 132 de la SEQ ID NO :12.

La présente invention a aussi pour objet, un polynucléotide isolé ou purifié codant pour un polypeptide chimérique selon l'invention.

La présente invention a aussi pour objet, un vecteur viral recombinant de la rougeole dans le génome duquel est inséré un polynucléotide selon l'invention.

La demande décrit aussi des anticorps monoclonaux ou polyclonaux purifiés reconnaissant spécifiquement au moins le polynucléotide défini précédemment et/ou le polypeptide chimérique de l'invention.

L'invention vise aussi l'utilisation d'un vecteur viral selon l'invention pour la préparation d'une composition immunogène destinée à la prévention ou au traitement d'une infection à *Flaviviridae* chez une espèce sensible.

La présente invention vise également des vecteurs de clonage ou d'expression comprenant un polynucléotide de l'invention.

Selon un autre objet, la présente invention propose une composition immunogène destinée à la prévention et/ou au traitement d'une infection à *Flaviviridae* chez une espèce sensible, caractérisée en ce qu'elle comprend au moins un des éléments suivants :
- un polypeptide chimérique tel que défini précédemment;
- un polynucléotide selon l'invention;
- un vecteur viral recombinant selon l'invention;
- un anticorps selon l'invention; et
- un vecteur de clonage et/ou d'expression selon l'invention.

La présente invention propose également une composition immunogène destinée à la prévention et/ou au traitement d'une infection à *Flaviviridae* chez une espèce sensible, comprenant au moins un des éléments suivants :
- un polypeptide chimérique tel que défini précédemment;
- un polynucléotide selon l'invention;
- un vecteur viral recombinant selon l'invention;
- un vecteur de clonage et/ou d'expression selon l'invention.

### BRÈVE DESCRIPTION DES FIGURES

La Figure 1 montre la séquence de 384 nucléotides (nt) codant le domaine [EDIII]_{DV1} de la souche virale FGA/89 fusionné par le dipeptide Arg-Ser avec le peptide signal de la calréticuline (ssCRT) encadrée des deux sites BsiWI (en 5') et BssHII (en 3') nécessaires à l'insertion dans le vecteur MV_{Schw}.
La Figure 2 montre la séquence de 516 nucléotides (nt) codant la protéine de fusion [EDIII + M¹⁻⁴⁰]_{DV1} de la souche virale FGA/89 fusionné par le dipeptide Arg-Ser avec le peptide signal de la calréticuline (ssCRT) encadrée des deux sites BsiWI (en 5') et BssHII (en 3') nécessaires à l'insertion dans le vecteur MV_{Schw}.
La Figure 3 montre des cellules Véro infectées avec des vecteurs viraux recombinants de la rougeole selon l'invention. Multiplicité d'infection de 10 TCIP50 /cellule pour 30 h. Immunofluorescence réalisées avec un anticorps spécifique anti-DV1 HMAF.
La Figure 4 montre l'expression et la sécrétion des domaines antigéniques [EDIIII_{DV-1} et [EDIII+M¹⁻⁴⁰]_{DV-1} dans les lysats cytoplasmiques (C) et les surnageants (S) de cellules Vero infectées par les virus recombinés MVSchw-DV-1
La Figure 5 montre l'expression et la sécrétion des domaines antigéniques [EDIIII_{DV-1} et [EDIII+M¹⁻⁴]_{DV-1} dans les sumageants filtrés et concentrés de cellules de drosophile S2 exprimant de manière inductible ces antigènes.

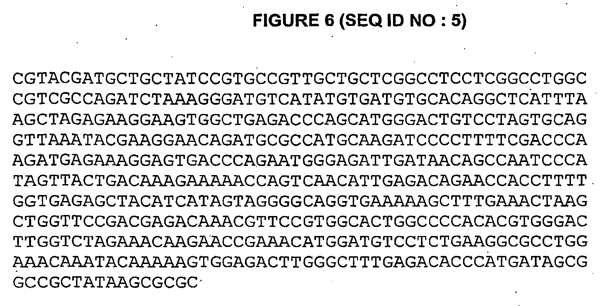

La Figure 6 montre une séquence nucléotidique codant pour un polypeptide chimérique selon un mode préféré de l'invention. -

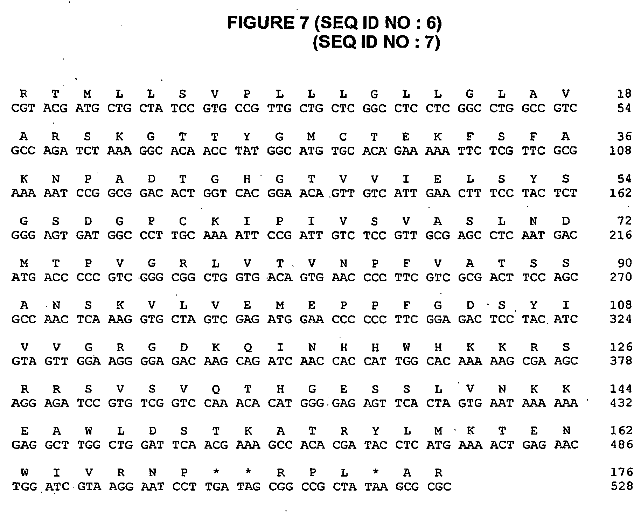

La Figure 7 montre une séquence en acides aminés d'un polypeptide chimérique selon un mode préféré de l'invention, ainsi que la séquence nucléotidique le codant.
La Figure 8 montre une séquence en acides aminés d'un dimère de l'ectodomaine III (EDIII) selon un mode préféré de l'invention, ainsi que la séquence nucléotidique le codant.
La Figure 9 montre une séquence en acides aminés d'un dimère de l'ectodomaine III (EDIII) selon un mode préféré de l'invention, ainsi que la séquence nucléotidique le codant.

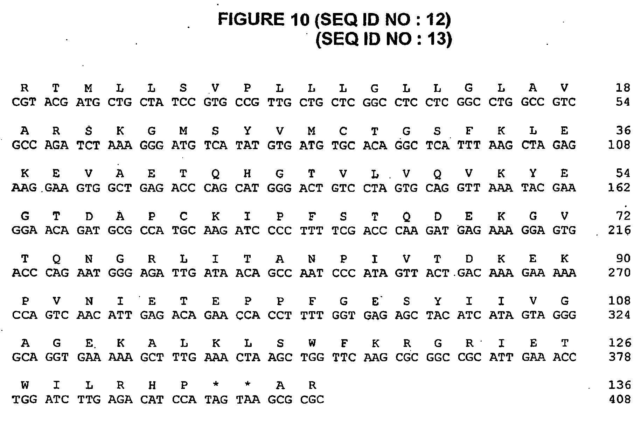

La Figure 10 montre une séquence en acides aminés d'un polypeptide chimérique selon un mode préféré de l'invention, ainsi que la séquence nucléotidique le codant.

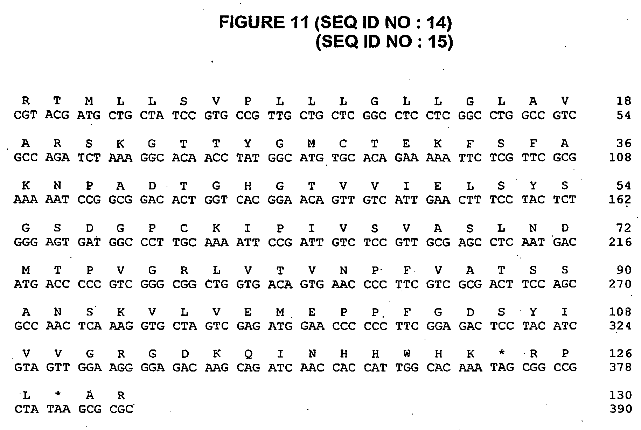

La Figure 11 montre une séquence en acides aminés de l'ectodomaine III (EDIII) selon un mode préféré de l'invention, ainsi que la séquence nucléotidique le codant.
La Figure 12 montre une séquence en acides aminés d'un dimère de l'ectodomaine III (EDIII) selon un mode préféré de l'invention, ainsi que la séquence nucléotidique le codant.

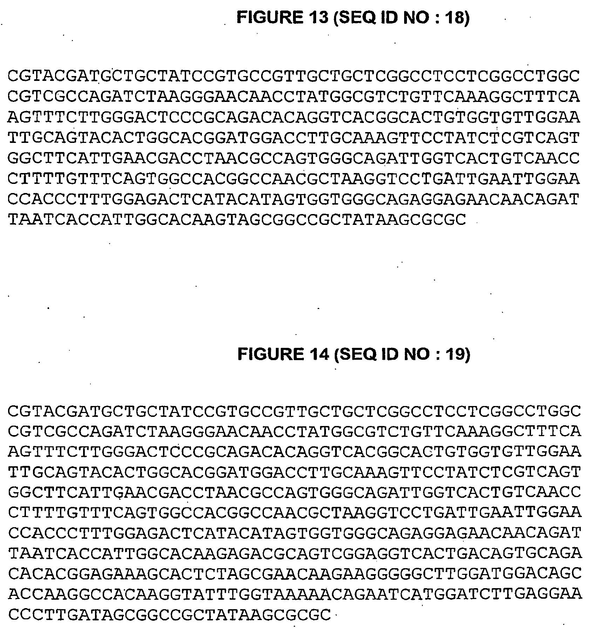

La Figure 13 montre une séquence en acides nucléiques codant pour un peptide de l'ectodomaine III (EDIII) selon un mode préféré de l'invention.
La Figure 14 montre une séquence nucléotidique codant pour un polypeptide chimérique selon un mode préféré de l'invention.
Les Figures 15 à 19 montrent une séquence d'acides aminés de polypeptides chimériques selon un mode préféré de l'invention.
Les Figures 20 à 24 montrent une séquence nucléotidique codant respectivement pour les polypeptides chimériques illustrés aux figures 15 à 19.
Les Figures 25 à 29 montrent respectivement un schéma représentatif des polypeptides chimériques des figures 15 à 19.
La Figure 30 montre la séquence peptidique de la séquence apoptoM de quatre sérotypes du virus de la Dengue utilisée dans la construction d'un polypeptide chimérique selon l'invention.

### RESCRIPTION DÉTAILLÉE DE L'INVENTION

L'originalité de la présente invention porte sur la construction d'un polypeptide chimérique et ses applications dans la prévention ou dans le traitement d'une infection à *Flaviviridae.* Par « *Flaviviridae* » on entend un virus sélectionné dans le groupe constitué par les virus du Nil Occidental, de la Dengue, de 'encéphalite japonaise et de la fièvre jaune.

### 1. Polypeptide et polynucléotide

Selon un premier aspect, la présente invention vise un polypeptide chimérique comprenant un peptide de sous-domaine de la protéine E de *Flaviviridae* lié à un peptide de sous-domaine de la protéine de membrane M de *Flaviviridae* et, le cas échéant, un segment de liaison liant le peptide de sous-domaine de la protéine E au peptide de sous-domaine de la protéine M, dans lequel le peptide de sous-domaine de la protéine M consiste en:
- l'ectodomaine 1-40 comprenant une séquence en acides aminés allant de la position 123 à 162 de la SEQ ID NO : 3; ou
- la séquence apoptoM comprenant une séquence en acides aminés allant de la position 154 à 162 de la SEQ ID NO : 3 ou allant de la position 122 à 132 de la SEQ ID NO :12. L'invention décrit aussi un polypeptide qui consiste en les susdits peptides.

Par « polypeptide » on entend, au sens de la présente invention, désigner indifféremment des protéines ou des peptides. Par « polypeptide chimérique » on entend toute protéine ou peptide comprenant des sous-parties de différentes origines, par exemple un polypeptide A provenant d'une première espèce et un polypeptide (protéine ou peptide) provenant d'une deuxième espèce. Une protéine ou un peptide est également considéré comme un polypeptide chimérique s'il comprend des sous-parties provenant de différentes protéines ou peptides d'une même espèce, ou bien d'une même protéine ou peptide de différentes espèces.

Préférablement, le peptide de sous-domaine de la protéine E consiste en l'ectodomaine III comprenant une séquence en acides aminés telle que définie dans l'une quelconque des séquences suivantes :
- acides aminés 18 à 120 de la SEQ ID NO : 20;
- acides aminés 171 à 273 de la SEQ ID NO : 20;
- acides aminés 324 à 426 de la SEQ ID NO : 20;
- acides aminés 477 à 579 de la SEQ ID NO : 20.

La demande décrit le peptide de sous-domaine de la protéine E consiste en un dimère de l'ectodomaine III des virus de la Dengue 1, 2, 3 ou 4 comprenant une séquence eh acides aminés telle que définie dans l'une quelconque des séquences suivantes :
- acides aminés 21 à 262 de la SEQ ID NO: 8; et
- acides aminés 21 à 262 de la SEQ ID NO : 10.

L'invention concerne en particulier un polypeptide chimérique dans lequel lé peptide du sous-domaine de la protéine consiste en un tétramère de l'ectodomaine III des virus de la Dengue 1, 2, 3 et 4 comprenant une séquence allant de l'acide aminé 18 à 429 de la SEQ ID NO :24.

En ce qui concerne le peptide de sous-domaine de la protéine M, celui-ci consiste en particulier en l'éctodomaine 1-40 comprenant une séquence en acides aminés allant de la position 123 à 162 de la SEQ ID NO : 3 ou en la séquence *apoptoM* comprenant une séquence en acides aminés allant de la position 154 à 162 de la SEQ ID NO: 3 ou allant de la position 122 à 132 de la SEQ ID NO :12.

Selon un mode préféré, le polypeptide chimérique de l'invention comprend en outre un segment de liaison liant le peptide de sous-domaine de la protéine E au peptide de sous-domaine de la protéine M. Ledit segment de liaison est préférablement un pentapeptide ayant pour séquence : RRDKR ou RREKR.

D'une manière très préférentielle, le polypeptide chimérique selon l'invention comprend une séquence en acides aminés telle que définie dans l'une quelconque des séquences suivantes :
- acides aminés 18 à 624 de la SEQ ID NO : 20;
- acides aminés 18 à 609 de la SEQ ID NO : 21;
- acides aminés 18 à 624 de la SEQ ID NO : 22;
- acides aminés 18 à 489 de la SEQ ID NO : 23; et
- acides aminés 21 à 474 de la SEQ ID NO : 24.

L'invention vise aussi les polypeptides (et les fragments de ceux-ci) qui sont codés par les séquences nucléotidiques mentionnées ci-après.

La demande divulgue un polypeptide qui présente un pourcentage d'identité d'au moins 80 % après alignement optimal avec une séquence telle que définie dans l'une quelconque des séquences d'acides aminés définies ci-dessus, de préférence d'au moins 90 %, plus préférentiellement d'au moins 98% et de façon la plus préférée d'au moins 100 %.

Selon un aspect connexe, l'invention vise un polynucléotide isolé ou purifié codant pour un polypeptide chimérique tel que défini précédemment.

Par "isolé ou purifié", on entend les molécules qui ont été altérées par l'homme de leur état natif, c'est-à-dire, si de telles molécules existent dans la nature, qu'elles ont été changées et/ou retirées de leur environnement initial. Par exemple, un polynucléotide ou un polypeptide naturellement présent et se trouvant dans l'environnement biologique de l'organisme vivant qui l'exprime naturellement n'est pas, dans ce contexte "isolé". Toutefois, le même polynucléotide ou polypeptide lorsque séparé de son environnement naturel et/ou obtenu par clonage, amplification et/ou par synthèse chimique est considéré selon la présente invention comme étant "isolé". Par ailleurs, un polynucléotide ou polypeptide qui est introduit dans un organisme par transformation, manipulation génétique ou par n'importe quelle autre méthode de recombinaison est "isolé" même s'il est présent dans ledit organisme.

Par séquence nucléotidique, acide nucléique, séquence nucléique ou d'acide nucléique, polynucléotide, oligonucléotide, séquence de polynucléotide, termes qui seront employés indifféremment dans la présente description, on entend désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin que des produits de transcription desdits ADNs. Ainsi, les séquences nucléiques selon l'invention englobent également les PNA (Peptid Nucleic Acid), ou analogues. Les fragments des polynucléotides de l'invention comprennent au moins 15 nucléotides consécutifs. Préférentiellement, ils comprennent au moins 20 nucléotides consécutifs et encore plus préférentiellement, ils comprennent au moins 30 nucléotides consécutifs.

L'invention a aussi pour objet des fragments de polynucléotides qui consistent en des fragments de 15, 20 ou 30 nucléotides successifs.

Tout polynucléotide qui a été modifié chimiquement, enzymatiquement ou métaboliquement mais qui a conservé les propriétés biochimiques du polypeptide chimérique d'origine est inclus dans la portée de la présente invention.

Selon un mode de réalisation préférentiel, le polynucléotide de la présente l'invention, lorsqu'il code pour un polypeptide chimérique selon l'invention, comprend avantageusement une séquence nucléotidique telle que définie dans l'une quelconque des séquences suivantes :
SEQ ID NOS : 25 à 29.

La demande décrit aussi les séquences suivantes :
a) nucléotides 7 à 492 de la SEQ ID NO : 4;
b) SEQ ID NO : 5;
c) nucléotides 7 à 504 de la SEQ ID NO : 7;
d) nucléotides 7 à 504 de la SEQ ID NO 13.

La demande divulgue aussi un polynucléotide qui présente un pourcentage d'identité d'au moins 80 % après alignement optimal avec une séquence telle que définie dans l'une quelconque des séquences nucléotidiques définies ci-dessus, de préférence d'au moins 90 %, plus préférentiellement d'au moins 98% et de façon la plus préférée d'au moins 100 %.

Par pourcentage d'identité entre deux séquences d'acides nucléiques ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. On entend désigner par "meilleur alignement" ou "alignement optimal", l'alignement pour lequel le pourcentage d'identité déterminé comme ci-après est le plus élevé. Les comparaisons de séquences entre deux séquences d'acides nucléiques ou d'acides aminés sont traditionnellement réalisées en comparant ces séquences après les avoir alignées de manière optimale, ladite comparaison étant réalisée par segment ou par fenêtre de comparaison pour identifier et comparer les régions locales de similarité de séquence. L'alignement optimal des séquences pour la comparaison peut être réalisé, outre manuellement, au moyen de l'algorithme d'homologie locale de Smith et Waterman (1981), au moyen de l'algorithme d'homologie locale de Neddleman et Wunsch (1970), au moyen de la méthode de recherche de similarité de Pearson et Lipman (1988), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI). Afin d'obtenir l'alignement optimal, on utilise de préférence le programme BLAST, avec la matrice BLOSUM 62. On peut également utiliser les matrices PAM ou PAM250.

Le pourcentage d'identité entre deux séquences d'acides nucléiques ou d'acides aminés est déterminé en comparant ces deux séquences alignées des manière optimale, la séquence d'acides nucléiques ou d'acides aminés à comparer pouvant comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions comparées et en multipliant le résultat obtenu par 100 pour obtenir le pourcentage d'identité entre ces deux séquences.

Par séquences nucléiques présentant un pourcentage d'identité d'au moins 80 %, de préférence d'au moins 90 %, de façon plus préférée d'au moins 98 %, après alignement optimal avec une séquence de référence, on entend désigner les séquences nucléiques présentant, par rapport à la séquence nucléique de référence, certaines modifications comme en particulier une délétion, une troncation, un allongement, une fusion chimérique, et/ou une substitution, notamment ponctuelle, et dont la séquence nucléique présente au moins 80 %, de préférence au moins 90 %, de façon plus préférée au moins 98 %, d'identité après alignement optimal avec la séquence nucléique de référence. De préférence, les conditions d'hybridation spécifiques ou de forte stringence seront telles qu'elles assurent au moins 80 %, de préférence au moins 90 %, de façon plus préférée au moins 98 % d'identité après alignement optimal entre l'une des deux séquences et la séquence complémentaire de l'autre.

Une hybridation dans des conditions de forte stringence signifie que les conditions de température et de force ionique sont choisies de telle manière qu'elles permettent le maintien de l'hybridation entre deux fragments d'acides nucléiques complémentaires. A titre illustratif, des conditions de forte stringence de l'étape d'hybridation aux fins de définir les séquences nucléotidiques décrites ci-dessus, sont avantageusement les suivantes.

L'hybridation ADN-ADN ou ADN-ARN est réalisée en deux étapes : (1) préhybridation à 42°C pendant 3 heures en tampon phosphate (20 mM, pH 7, 5) contenant 5 x SSC (1 x SSC correspond à une solution 0, 15 M NaCl + 0, 015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation proprement dite pendant 20 heures à une température dépendant de la taille de la sonde (i. e. :42°C, pour une sonde de taille > 100 nucléotides) suivie de 2 lavages de 20 minutes à 20°C en 2 x SSC + 2 % SDS, 1 lavage de 20 minutes à 20 C en 0,1 x SSC + 0,1 % SDS. Le dernier lavage est pratiqué en 0,1 x SSC + 0,1 % SDS pendant 30 minutes à 60°C pour une sonde de taille > 100 nucléotides. Les conditions d'hybridation de forte stringence décrites ci-dessus pour un polynucléotide de taille définie peuvent être adaptées par l'homme du métier pour des oligonucléotides de taille plus grande ou plus petite, selon l'enseignement de Sambrook et al., 1989.

Les polypeptides et polynucléotides selon la présente invention peuvent être préparés par tout procédé approprié. Ils peuvent notamment être obtenus par synthèse chimique mais il est également possible de les obtenir par voie biologique en utilisant notamment différents vecteurs dans les cultures cellulaires appropriées. Les peptides selon la présente invention peuvent se présenter sous forme déglycosylée, ou glycosylée, si cela est nécessaire. Une personne versée dans le domaine de l'invention saura obtenir différents polynucléotides/polypeptides et elle saura également déterminer quels sont, parmi les polynucléotides/polypeptides obtenus, ceux qui ont une activité biologique adéquate.

### 2. Vecteur viraux recombinants

Selon un autre aspect, l'invention concerne un vecteur viral recombinant de la rougeole dans le génome duquel est inséré un polynucléotide selon l'invention. De tels vecteurs sont préparés selon les méthodes couramment utilisées par l'homme du métier, et les clones en résultant peuvent être introduits dans un hôte approprié par des méthodes standard connues par l'homme de l'art.

Selon un mode privilégié, le vecteur viral recombinant selon l'invention est avantageusement un vecteur viral vivant rougeole souche Schwarz, comme ceux sélectionnés dans le groupe de vecteurs viraux constitué par ceux déposés à la CNCM sous les numéros I-3440, I-3442, I-3452, I-3453, I-3454, I-3455, I-3619, I-3620, I-3621, I-3622 et I-3623. La séquence de l'antigénome complet du virus de la rougeole souche Schwarz peut être obtenue par exemple à partir des plasmides pTM des souches déposées sous les numéros CNCM I-3440 et I-3442.

Le vecteur pTM-MVSchw2-[EDIII+M¹⁻⁴⁰]WNV(IS-98-ST1) a été déposé à la CNCM (Paris, France) le 26 Mai 2005 sous le numéro I-3440.

Ce vecteur est un plasmide PTM contenant l'antigénome complet du virus de la rougeole, souche Schwarz, et une unité additionnelle d'expression placée entre les gènes P et M contenant la séquence du domaine III de la protéine d'enveloppe du virus du Nil occidental (WNV IS-98-ST1) fusionnée à la séquence 1-40 de la protéine de membrane.

Le vecteur peut être cultivé en milieu LB ampi 100 µ/ml après ensemencement par de petites colonies.

Il est conservé à long terme par congélation à -80°C.

Le vecteur pTM-MVSchw2-[EDIII+ApoptoM]DV1(FGA89) a été déposé à la CNCM (Paris, France) le 26 Mai 2005 sous le numéro I-3442.

Ce vecteur est un plasmide pTM contenant l'antigénome complet du virus de la rougeole, souche Schwarz, et une unité additionnelle d'expression placée entre les gènes P et M contenant la séquence du domaine III de la protéine d'enveloppe du virus de la dengue 1, souche FGA89, fusionnée à la séquence apoptotique de la protéine de membrane M.

Le vecteur peut être cultivé en milieu LB ampi 100 µ/ml après ensemencement par de petites colonies.

Il est conservé à long terme par congélation à -80°C.

Le vecteur pTRE₂-SSCRT-ApoptoMden1/DIII-Hα1-2 a été déposé à la CNCM (Paris, France) le 16 Juin 2006 sous le numéro I-3452.

Ce vecteur est un plasmide pTRE₂ contenant la séquence du peptide signal de la calbréticuline humaine fusionnée aux séquences proapoptotiques de la protéine M et aux ectodomaines III de la protéine E avec les séquences hélice α des virus de la dengue 1 et 2.

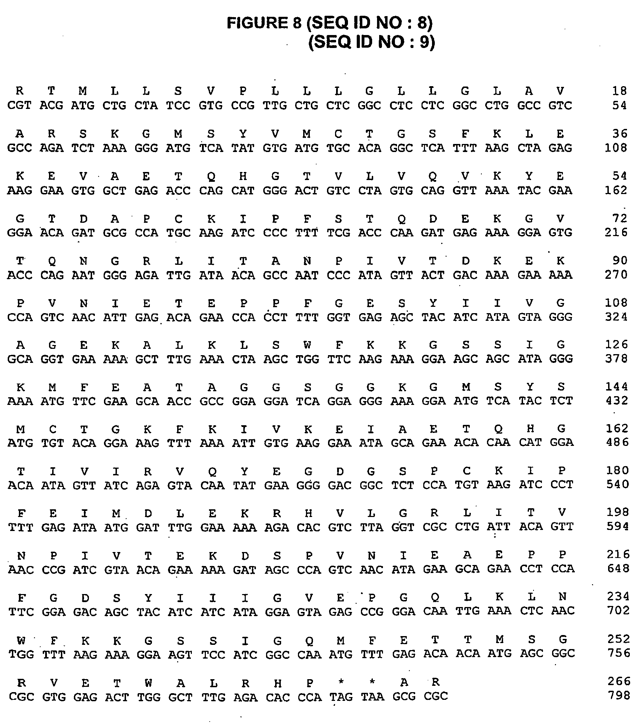

La séquence de l'insert contenu dans le plasmide et constituant un polynucléotide de l'invention, est la SEQ ID NO : 9 représentée à la figure 8.

Le vecteur peut être cultivé en milieu LB ampi 100 µ/ml.

Il est conservé à long terme par congélation à -80°C.

Le vecteur pTRE₂-ssCRT-ApoptoMden1/DIII-Hα3-4 a été déposé à la CNCM (Paris, France) le 16 Juin 2005 sous le numéro I-3453.

Ce vecteur est un plasmide pTRE2 contenant la séquence du peptide signal de la calbréticuline humaine fusionnée aux séquences proapoptotique de la protéine M du virus de la dengue 1 et aux séquences des ectodomaines III et Hα des virus de la dengue 3-4.

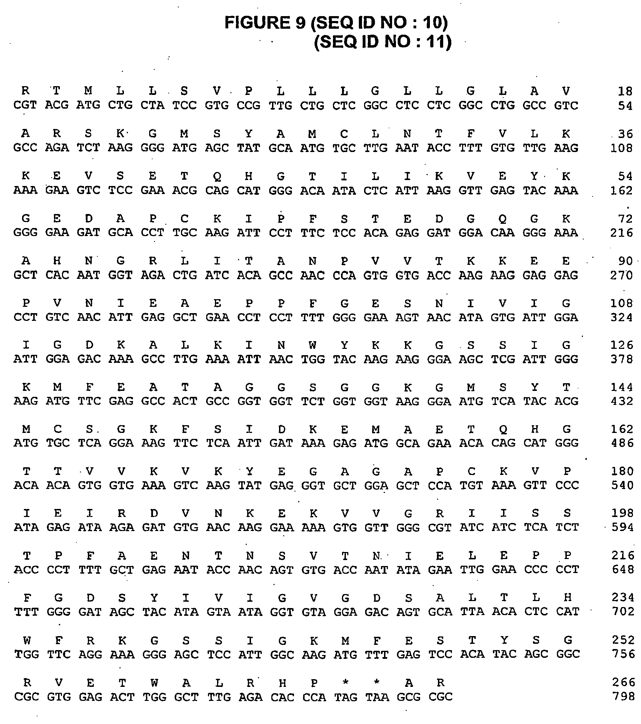

La séquence de l'insert contenu dans le plasmide et constituant un polynucléotide de l'invention, est la SEQ ID NO : 11 représentée à la figure 9.

Le vecteur peut être cultivé en milieu LB ampi 100 µ/ml.

Il est conservé à long terme par congélation à -80°C.

Le vecteur pTRE₂-ssCRT-ApoptoMden1/DIII-Hα1-2-3-4 a été déposé à la CNCM (Paris, France) le 16 Juin 2005 sous le numéro I-3454.

Ce vecteur est un plasmide pTRE₂ contenant la séquence du peptide signal de la calbréticuline humaine fusionnée aux séquences proapoptotiques de la protéine M du virus de la dengue 1 et aux séquences des ectodomaines III avec hélices α(Hα) des protéines E des virus de la dengue 1, 2, 3, 4.

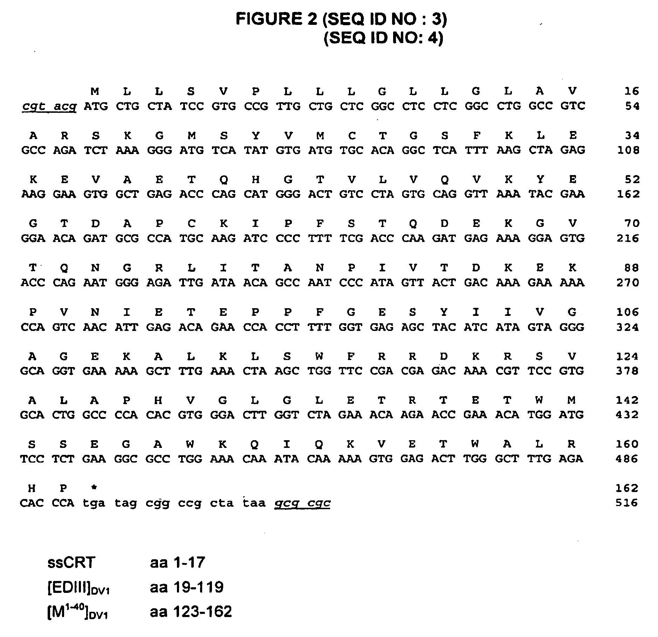

La séquence de l'insert contenu dans le plasmide et constituant un polynucléotide de l'invention, est la SEQ ID NO : 4 représentée à la figure 2.

Le vecteur peut être cultivé en milieu LB ampi 100 µ/ml.

Il est conservé à long terme par congélation à -80°C.

Le vecteur pTRE₂-ssCRT-EctoM40den1/DIII-Hα1-2-3-4 a été déposé à la CNCM (Paris, France) le 16 Juin 2005 sous le numéro I-3455.

Ce vecteur est un plasmide pTRE2 contenant la séquence du peptide signal de la calbréticuline humaine fusionnée aux séquences de l'ectodomaine 1-40 de la protéine M du virus de la dengue 1 et aux séquences des ectodomaines III avec hélices α(Hα) des protéines E des virus de la dengue 1, 2, 3, 4.

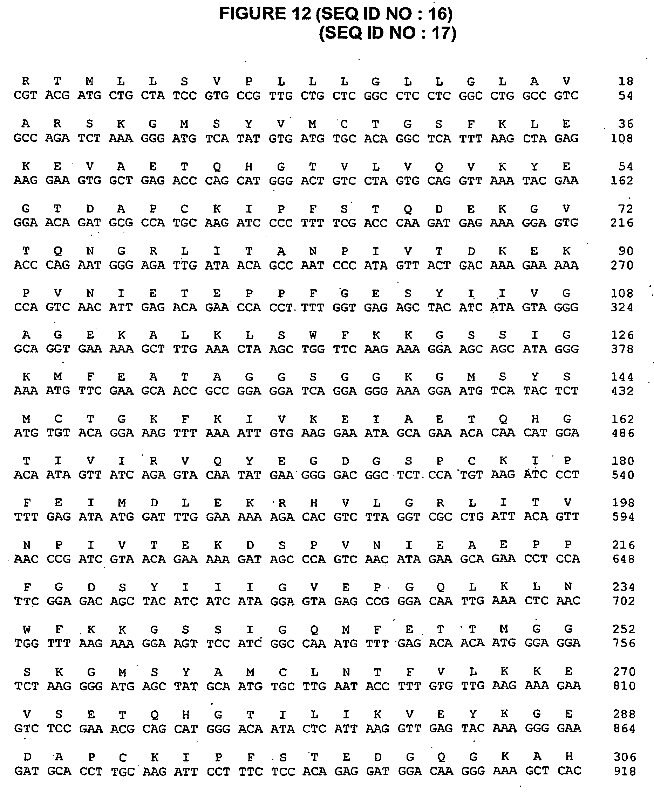

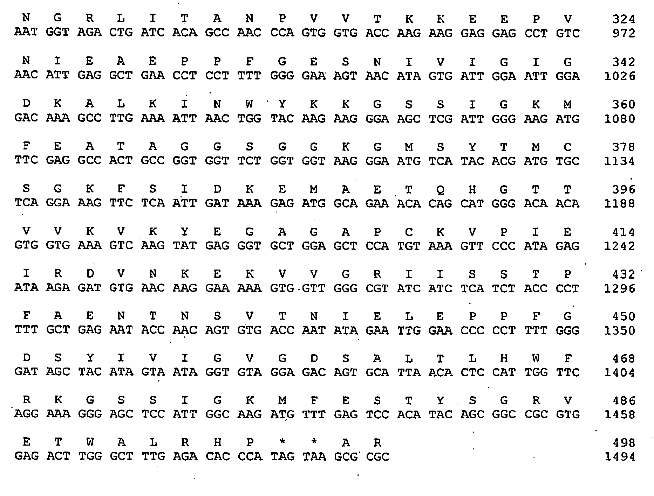

La séquence de l'insert contenu dans le plasmide et constituant un polynucléotide de l'invention, est la SEQ ID NO : 17 représentée à la figure 12.

Le vecteur peut être cultivé en milieu LB ampi 100 µ/ml.

Il est conservé à long terme par congélation à -80°C.

Le vecteur pUC57-TetraDVA (introduit dans une souche *E. coli*) a été déposé à la CNCM (Paris-France) le 14 juin 2006 sous le numéro I-3619.

Ce vecteur est un plasmide pUC contenant une séquence nucléotidique optimisée pour l'expression en cellules mammifères d'une construction tétramérique des ectodomaines III de la protéine d'enveloppe E des 4 sérotypes (1, 2, 3, 4) du virus de la dengue, fusionnée avec l'ectodomaine de la protéine de membrane M.

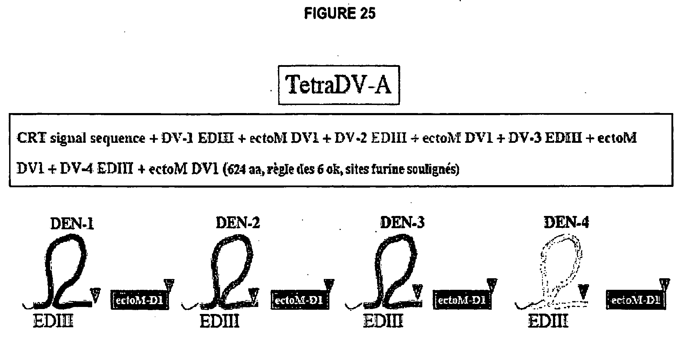

L'insert contenu dans le plasmide vecteur est représenté à la figure 25.

La souche contenant le vecteur peut être cultivée en milieu LB ampi 100 µg/ml.

Le vecteur pUC57-TetraDVB (introduit dans une souche *E. coli*) a été déposé à la CNCM (Paris-France) le 14 juin 2006 sous le numéro I-3620.

Ce vecteur est un plasmide pUC contenant une séquence nucléotidique optimisée pour l'expression en cellules mammifères d'une construction tétramérique des ectodomaines III de la protéine d'enveloppe E des 4 sérotypes (1, 2, 3, 4) du virus de la dengue, fusionnée avec l'ectodomaine de la protéine de membrane M.

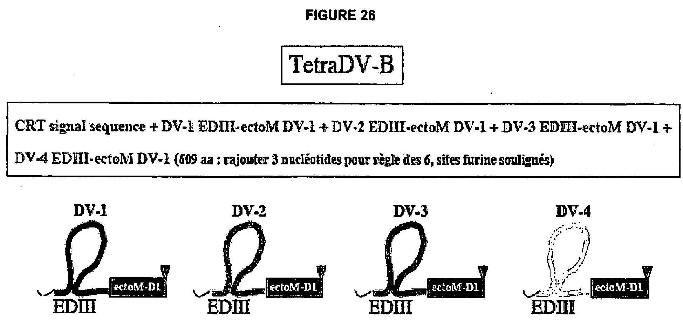

L'insert contenu dans le plasmide vecteur est représenté à la figure 26.

La souche contenant le vecteur peut être cultivée en milieu LB ampi 100 µg/ml.

Le vecteur pUC57-TetraDVC (introduit dans une souche *E. coli*) a été déposé à la CNCM (Paris-France) le 14 juin 2006 sous lé numéro 1-3621.

Ce vecteur est un plasmide pUC contenant une séquence nucléotidique optimisée pour l'expression en cellules mammifères d'une construction tétramérique des ectodomaines III de la protéine d'enveloppe E des 4 sérotypes (1, 2, 3, 4) du virus de la dengue, fusionnée avec l'ectodomaine de la protéine de membrane M.

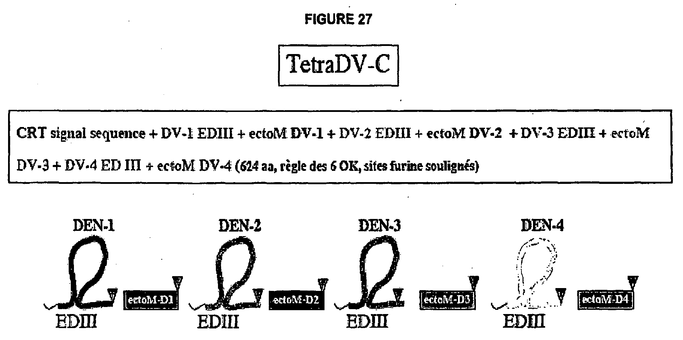

L'insert contenu dans le plasmide vecteur est représenté à la figure 27.

La souche contenant le vecteur peut être cultivée en milieu LB ampi 100 µg/ml.

Le vecteur pUC57-TetraDVD (introduit dans une souche *E. coli*) a été déposé à la CNCM (Paris-France) le 14 juin 2006 sous le numéro I-3622.

Ce vecteur est un plasmide pUC contenant une séquence nucléotidique optimisée pour l'expression en cellules mammifères d'une construction tétramérique des ectodomaines III de la protéine d'enveloppe E des 4 sérotypes (1, 2, 3, 4) du virus de la dengue, fusionnée avec l'ectodomaine de la protéine de membrane M.

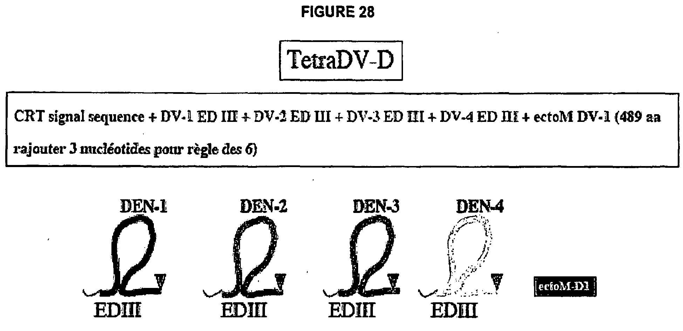

L'insert contenu dans le plasmide vecteur est représenté à la figure 28.

La souche contenant le vecteur peut être cultivée en milieu LB ampi 100 µg/ml.

Le vecteur pUC57-TetraDVE (introduit dans une souche *E*. *coli*) a été déposé à la CNCM (Paris-France) le 14 juin 2006 sous le numéro I-3623.

Ce vecteur est un plasmide pUC contenant une séquence nucléotidique optimisée pour l'expression en cellules mammifères d'une construction tétramérique des ectodomaines III de la protéine d'enveloppe E des 4 sérotypes (1, 2, 3, 4) du virus de la dengue, fusionnée avec l'ectodomaine de la protéine de membrane M.

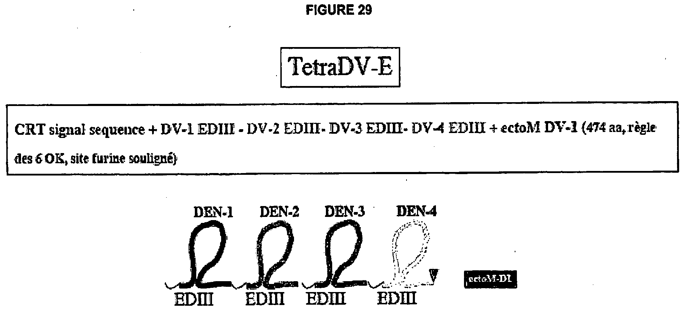

L'insert contenu dans le plasmide vecteur est représenté à la figure 29.

La souche contenant le vecteur peut être cultivée en milieu LB ampi 100 µg/ml.

### 3. Vecteurs de clonage et/ou d'expression et anticorps

Selon un autre aspect, l'invention concerne tout vecteur de clonage et/ou d'expression et tout hôte cellulaire (procaryote ou eucaryote) transformé par un tel vecteur, et comprenant les éléments de régulation permettant l'expression de la séquence de nucléotides codant pour un polypeptide chimérique selon l'invention. De tels vecteurs sont préparés selon les méthodes couramment utilisées par l'homme du métier, et les clones en résultant peuvent être introduits dans un hôte approprié par des méthodes standard, telles que par exemple la lipofection, l'électroporation, le choc thermique, la transformation après perméabilisation chimique de la membrane, la fusion cellulaire.

La demande décrit aussi des cellules hôtes, notamment les cellules eucaryotes et procaryotes, transformées par les vecteurs selon l'invention ainsi que les animaux transgéniques, de préférence les mammifères, excepté l'homme, comprenant une desdites cellules transformées selon l'invention. Ces animaux peuvent être utilisés eh temps que modèles, pour l'étude de l'étiologie de maladies inflammatoires et/ou immunes, et en particulier des maladies inflammatoires du tube digestif, ou pour l'étude de cancers.

Parmi les cellules utilisables, on peut citer les cellules bactériennes (Olins et Lee (1993), Curr. Op. Biotechnology 4 : 520), mais aussi les cellules de levure (Buckholz, (1993), Curr. Op. Biotechnology 4,538), de même que les cellules animales, en particulier les cultures de cellules de mammifères (Edwards et Aruffo (1993), Curr. Op. Biotechnology, 4,558).

Dans le contexte de la présente invention, le terme "vecteur de clonage et/ou d'expression" se réfère à une construction polynucléotidique conçue pour être transfectée dans différents types cellulaires. De ce fait ces vecteurs visent les vecteurs d'expression conçus pour l'expression d'une séquence nuctéotidique dans une cellule hôte; les vecteurs de clonage conçus pour l'isolement, la propagation et la réplication de nucléotides insérés ou des vecteurs navettes qui comprennent des attributs de plus d'un vecteur.

### 4. Anticorps polyclonaux ou monoclonaux

Les polypeptides et polynucléotides de la présente invention peuvent aussi être utilisés pour préparer des anticorps polyclonaux ou monoclonaux capables de se fixer (préférablement de manière spécifique) sur au moins un polypeptide chimérique/polynucléotide objet de l'invention. La demande décrit donc également de tels anticorps purifiés qui peuvent être obtenus par des techniques très bien connues comme par exemple la technique décrite par Kolher et Milstein (Continuous cultures of fused cells secreting antibody of predefined specificity, Nature (1975), 262:495-497). La demande divulgue des anticorps de type « humanisé ». Une personne versée dans le domaine de par ses connaissances générales saura comment préparer ces types d'anticorps.

### 5. Méthodes et Procédé d'utilisation

Selon un autre aspect, l'invention concerne la prévention et/ou le traitement d'une infection à *Flaviviridae* chez une espèce sensible. Plus particulièrement, l'invention vise l'utilisation d'un vecteur viral recombinant selon l'invention pour la préparation d'une composition immunogène destinée à la prévention ou au traitement d'une infection à *Flaviviridae* chez une espèce sensible. Par « espèce sensible », on entend tout animal susceptible, à une infection à *Flaviviridae,* comme par exemple un humain.

L'invention vise aussi une composition immunogène destinée à la prévention et/ou au traitement d'une infection à *Flaviviridae* chez une espèce sensible, comprenant au moins un des éléments suivants :
- un polypeptide chimérique selon l'invention;
- un polynucléotide selon l'invention;
- un vecteur viral recombinant selon ('invention;
- un vecteur de clonage et/ou d'expression selon l'invention.

Par « infection à *Flaviviridae »* on entend par exemple des flaviviroses telles la dengue, la fièvre jaune, l'encéphalite japonaise et la fièvre du Nil Occidental. Les moyens de préparation et d'administration des éléments de la présente invention ne seront pas décrits davantage car ceux-ci sont déjà connus par l'homme de l'art.

### 6. Compositions

La présente invention concerne également des compositions immunogènes utiles dans la prévention et/ou dans le traitement d'une infection à *Flaviviridae.* Par « composition immunogène » on entend une composition qui contient des éléments ayant la capacité d'induire in vivo ou in vitro, une réponse immune de type cellulaire et/ou humorale.

Dans un mode de réalisation préféré, la composition de la présente invention contient en outre un véhicule pharmaceutiquement acceptable, et un élément choisi dans le groupe constitué par :
- un polynucléotide selon la présente invention;
- un polypeptide chimérique selon la présente invention;
- un vecteur viral recombinant selon l'invention;
- un anticorps selon la présente invention; et
- un vecteur de clonage et/ou d'expression selon la présente invention.

Les compositions selon la présente invention peuvent se présenter sous une forme solide ou liquide quelconque habituelle pour l'administration pharmaceutique, c'est-à-dire par exemple des formes d'administration liquide, en gel, ou tout autre support permettant par exemple la libération contrôlée. Parmi les compositions utilisables, on peut citer notamment les compositions injectables chez l'humain.

Les compositions de l'invention peuvent comporter également des composants augmentant ou susceptibles d'augmenter l'immunogénicité des polypeptides chimériques de l'invention, notamment d'autres peptides immunogènes, des adjuvants d'immunité spécifiques ou non tels que l'alun, le QS21, l'adjuvant de Freund, l'adjuvant SBA₂, la montanide, des polysaccharides ou des composés équivalents.

Une personne versée dans le domaine saura préparer des compositions pharmaceutiquement acceptables et déterminer, en fonction de plusieurs facteurs, le mode d'administration privilégié et la quantité devant être administrée. Parmi les facteurs pouvant influencer ses choix l'on retrouve: la nature du traitement, la nature exacte des ingrédients, actifs ou non, entrant dans la composition; le stade de la maladie; la condition, l'âge et le poids du patient, etc.

### EXEMPLES

Les exemples ci-après permettront de mettre en évidence d'autres caractéristiques et avantages de la présente invention, et servent à illustrer l'étendue de l'utilisation de la présente invention et non à limiter sa portée. Des modifications et variations peuvent y être effectuées sans que l'on échappe à l'esprit et à la portée de l'invention. Bien que l'on puisse utiliser d'autres méthodes ou produits équivalents à ceux que l'on retrouve ci-dessous pour tester ou réaliser la présente invention, le matériel et les méthodes préférés sont décrits.

### Exemple 1 : Le virus de la rougeole recombiné MV_{Schw}-[EDIII + M¹⁻⁴⁰]DV-1 comme prototype d'un vaccin candidat contre la dengue (PTR156)

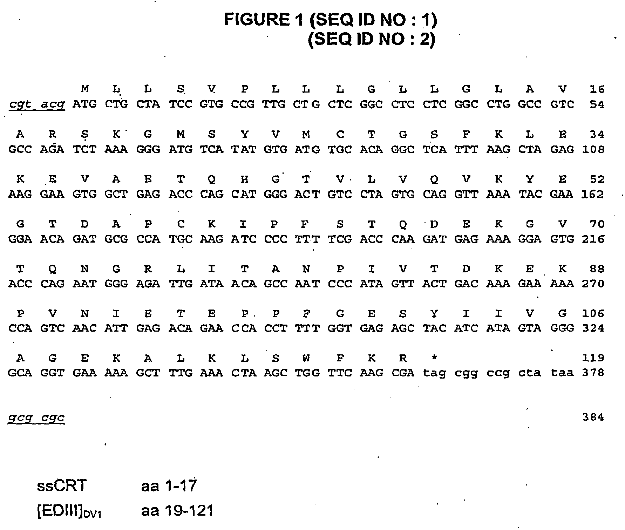

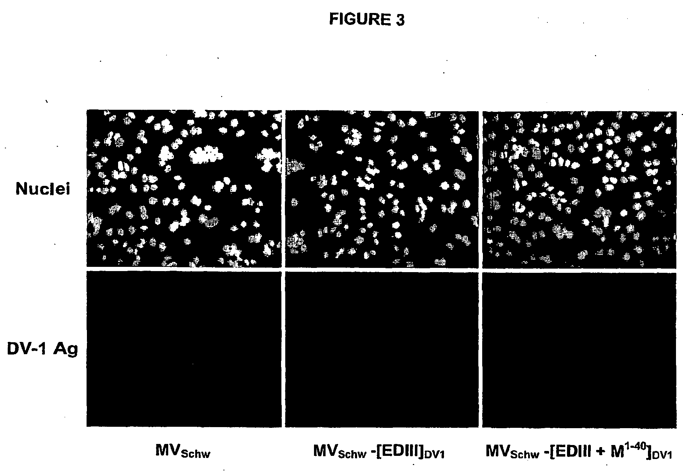

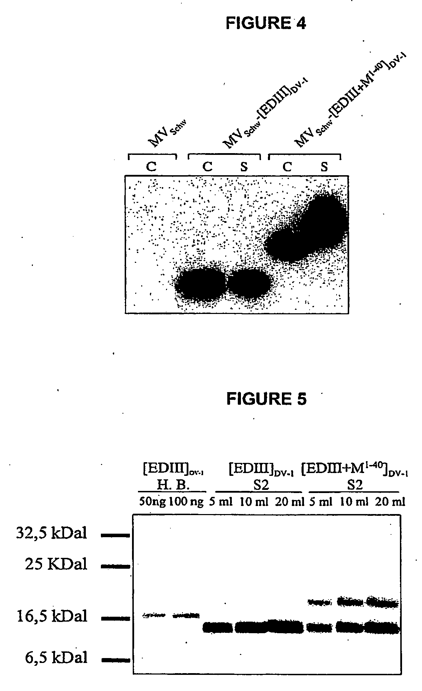

Décrites dans les Figures 1 et 2, deux constructions immunogéniques basées d'une part sur la capacité du domaine III de la glycoprotéine d'enveloppe E du virus de la dengue à induire des anticorps neutralisants et d'autre part sur l'implication de l'ectodomaine de la protéine de membrane M (M¹⁻⁴⁰) dans la pathogénicité virale (apoptose) ont été insérées dans le génome de la souche virale atténuée Schwarz du virus de la rougeole (MVSchw). Les séquences virales sont sous la dépendance du peptide signal de la calréticuline pour permettre leur adressage dans la voie de sécrétion. L'expression des séquences [EDIII]_{DV-1} et [EDIII+M¹⁻⁴⁰]_{DV-1} par les virus recombinés MVSchw a été vérifiée dans les cellules Vero infectées par immunofluorescence indirecte à l'aide d'un sérum polyclonal de souris HMAF dirigé contre le DV-1 (Fig. 3). La sécrétion des domaines antigéniques EDIII et EDIII fusionnés à la séquence M¹⁻⁴⁰ (avec le pentapeptide intercalant RRDKR) du virus dengue, type-1 (DV-1) de la souche FGA/89 (Holmes E.C. et al, Mol. Biol. Evol. 16(3) : 405-409, 1999 et Desprès P. et al, Virology 196 :209-219) a été observée par analyse en Western blot dans les surnageants de cellules infectées par les virus MV_{Schw}-[EDIII]_{DV-1} et MV_{Schw}-[EDIII+M¹⁻⁴⁰]_{DV-1}, respectivement (Fig. 4). De.même, la production et la sécrétion dans les surnageants de culture d'une lignée de cellules de drosophile S2/[EDIII+M¹⁻⁴⁰]_{DV-1} induites pour l'expression de ces protéines ont été démontrées (Fig. 5).

Deux groupes de 4 souris CD46/IFNAR adultes ont été immunisés avec 10⁴ TCID50 de chacun des virus MVSchw recombinés (Tableaux 1 et 2). Le virus vide MVSchw a été utilisé comme contrôle négatif. Nous avons déterminé la production d'anticorps dirigés contre le virus MVSchw (MV Ag) et le virus DV-1 (DV-1 Ag) y compris ceux qui sont spécifiques du EDIIII (Thullier, P. et al. 2001. J. Gen. Virol. 82 : 1885-1892) et neutralisants (Anti-DV-1 FRNT75) chez les souris immunisées (Tableaux 1 et 2). Nous observons que le virus MV_{Schw}-[EDIII]_{DV-1} est peu efficace à produire des anticorps spécifiques du DV-1 ou du seul EDIII après deux inoculations espacées d'un mois (Tableau 1). Selon le même protocole d'immunisation, nous observons que le virus MV_{Schw}-[EDIII+M¹⁻⁴⁰]_{DV-1} induit des titres significatifs d'anticorps dirigés contre le DV-1 et contre le seul EDIII (Tableau 2). Des anticorps anti-DV1 dont ceux qui sont neutralisants sont encore détectés quatre mois après la fin de l'immunisation.

Lorsque les souris immunisées plus de 6 mois avec MV_{Schw}-[EDIII+M¹⁻⁴⁰]_{DV-1} subissent un rappel avec une dose unique de 5 µg de protéines totales d'un concentrât de surnageant de cellules de drosophile S2/[EDIII+M¹⁻⁴⁰]_{DV-1} induites pour l'expression de la protéine de fusion [EDIII+M¹⁻⁴⁰]_{DV-1} et en présence d'Alugel comme adjuvant, des taux élevés d'anticorps anti-DV-1, anti-EDIII et neutralisants du DV-1 sont observés, qui traduisent une réponse humorale mémoire bien établie chez ces animaux vaccinés (Tableau 2). Les anticorps anti-DV-1 sont encore présents plusieurs mois après le rappel antigénique (Tableau 2).

Trois mois après le rappel antigénique avec r[EDIII+M¹⁻⁴⁰]_{DV-1}, les souris immunisées avec MV_{Schw}-[EDIII+M¹⁻⁴⁰]_{DV-1} ont été inoculées avec 10⁷ UFF du DV-1 souche FGA/NA d1d par la voie intrapéritonéale (le DV-1 est responsable d'une infection asymptomatique chez la souris IFNAR). Des titres importants en anticorps anti-DV-1 (surtout dirigés contre le EDIII) dont ceux qui neutralisent le DV-1 souche Hawaï (titre neutralisant # 4000) sont observés après 3 semaines d'épreuve virale (Tableau 2). À noter que les souris immunisées 9 mois avec le virus MV_{Schw}-[EDIII+M¹⁻³⁰]_{DV-1}, (cette construction n'induit pas d'anticorps anti-EDIII ou neutralisants du DV1) puis éprouvées avec 10⁷ UFF du DV-1 souche FGA/NA d1d par la voie intrapéritonéale, produisent des titres en anticorps anti-DV1, anti-EDIII et neutralisants de 20000, 5000 et 80, respectivement; ce sont des valeurs équivalentes à celles observées chez les souris BALB/c ou IFNAR éprouvées selon les mêmes conditions expérimentales.

En conclusion, la séquence de fusion [EDIII+M¹⁻⁴⁰]_{DV-1} sécrétée par le virus MV_{Schw}-[EDIII+M¹⁻⁴⁰]_{DV-1} est capable de générer des anticorps neutralisants anti-DV-1 et d'induire une réponse humorale mémoire sur le long terme qui est stimulée efficacement d'une part par l'antigène soluble r[EDIII+M¹⁻⁴⁰]_{DV-1} et d'autre part, en réponse à une infection virale. À l'inverse, le seul domaine antigénique EDIII sécrété par le virus MV_{Schw}-[EDIII)_{DV-1} est peu immunogène chez les souris CD46⁺-IFNAR. Nos travaux complémentaires démontrent que le peptide pro-apoptotique *ApoptoM* (M³²⁻⁴⁰) à l'extrémité C-terminale de l'ectodomaine 1-40 de la protéine M est déterminant dans le pouvoir immunogène de la séquence de fusion [EDIII+M¹⁻⁴⁰]_{DV-1} puisque le virus MV_{Schw}-[EDIII+M¹⁻³⁰]_{DV-1} sans *ApoptoM* induit une production d'anticorps anti-DV-1 équivalente à celle obtenue après immunisation avec le virus MV_{Schw}-[EDIII]_{DV-1}.

Comme méthodologie générale de vaccination pédiatrique contre la dengue, nous proposons d'immuniser les jeunes individus avec le virus MV_{Schw}-[EDIII+M¹⁻⁴⁰]_{DV-1} par une double inoculation espacée d'un mois, puis de les restimuler tardivement avec l'antigène soluble r[EDIII+M¹⁻⁴⁰]_{DV-1} dans un but de rappel vaccinal ou de prophylaxie contre le risque d'une infection par le virus de la dengue. Cette stratégie d'immunisation est en cours de validation pour les quatre sérotypes de la dengue sur la base d'un antigène composé du tétramère des 4 domaines EDIII des DV-1, -2, -3 et -4 fusionné à la séquence cytotoxique *ApoptoM.*

Nos premiers résultats expérimentaux soulignent l'importance d'un immunogène de taille réduite dérivé de la protéine d'enveloppe du virus DV1 en combinaison avec les capacités immunostimulatrices du vecteur rougeole vivant, une stratégie permettant l'induction d'une réponse humorale neutralisante qui est efficace contre le virus de la dengue. Ils définissent une preuve de concept pour le design des constructions tétramériques des domaines antigéniques du DV qui permettront d'immuniser simultanément et sur le long terme contre les quatre sérotypes de la dengue.

### Exemple 2 : Construction de polypeptides chimériques optimisés pour une expression en mammifère

Dans le présent exemple, les inventeurs ont développé de nouvelles constructions antigéniques utiles contre une infection à *Flaviviridae.*

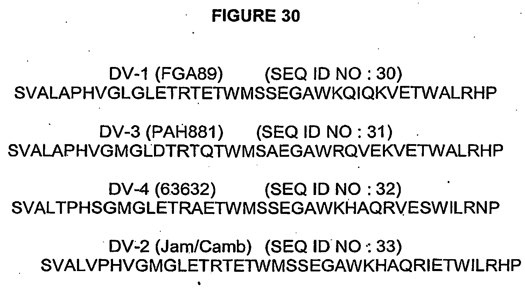

Ces nouveaux polypeptides chimériques sont basés sur un antigène composé d'un tétramère des quatre domaines EDIII des quatre sérotypes du virus de la Dengue, fusionnés entre eux et à l'une ou l'autre des séquences cytotoxiques apoptoM montrées à la Figure 30.

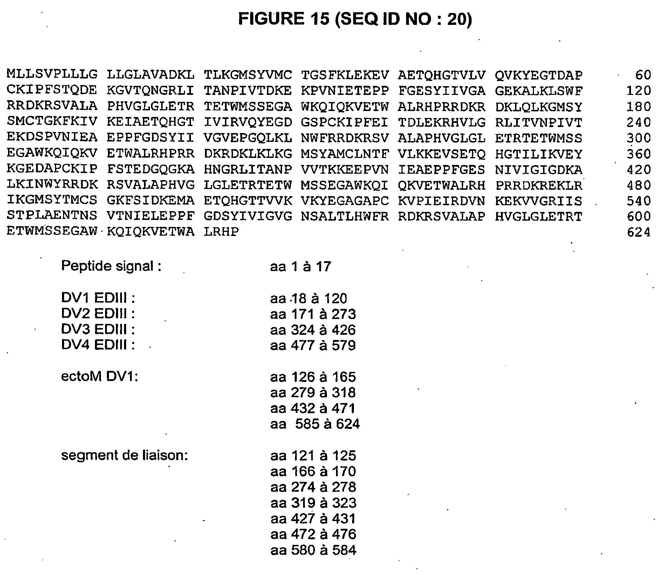

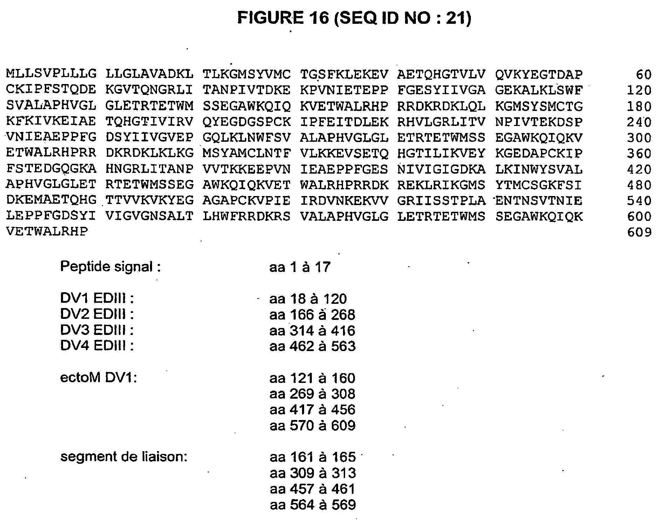

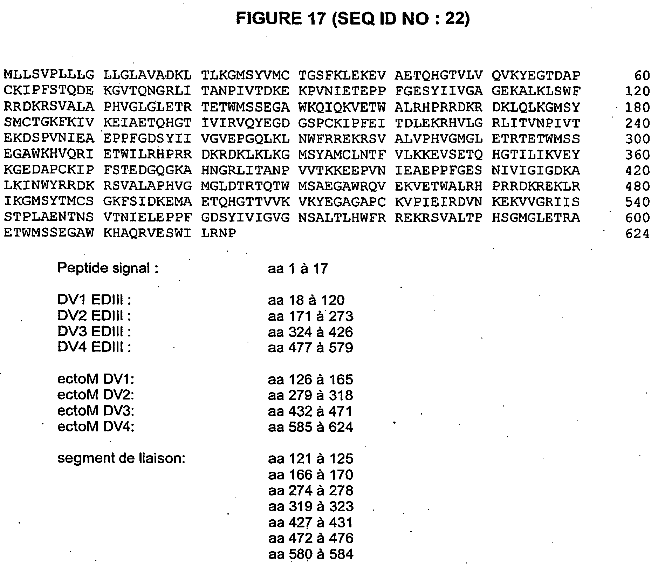

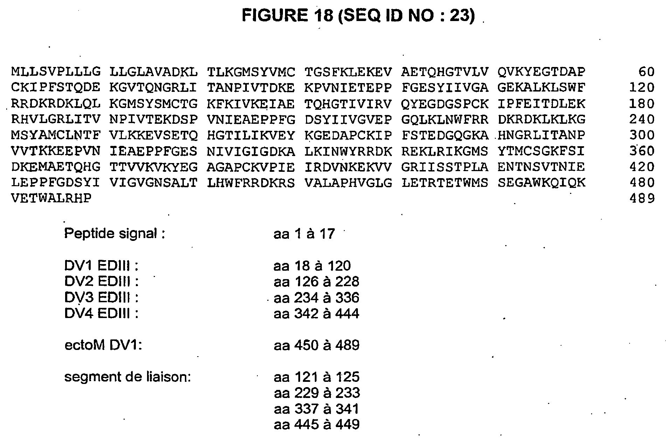

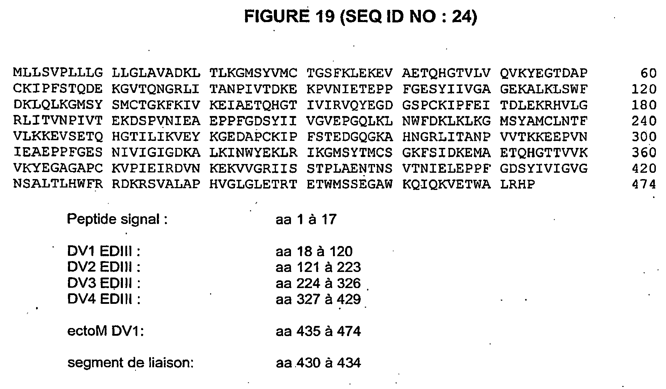

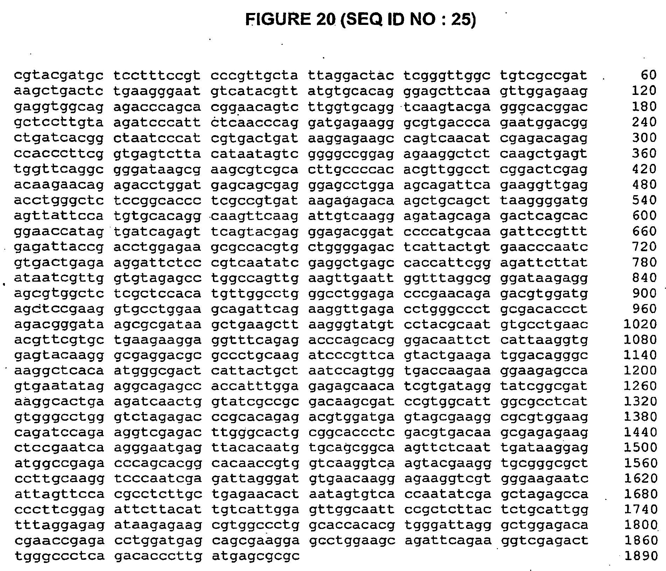

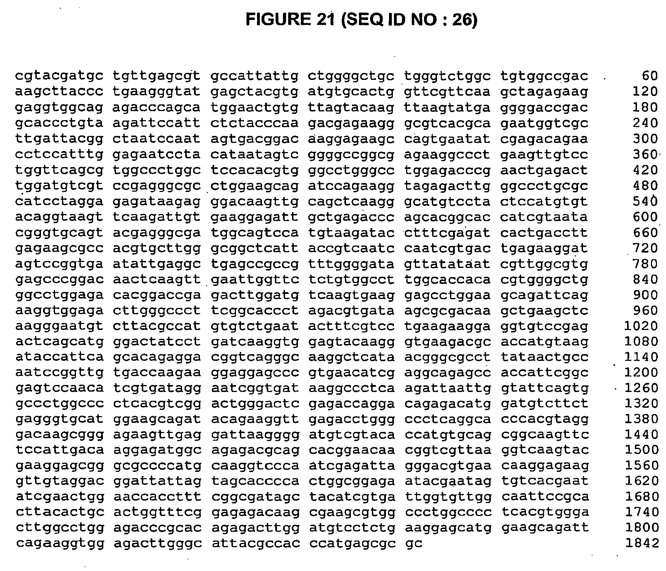

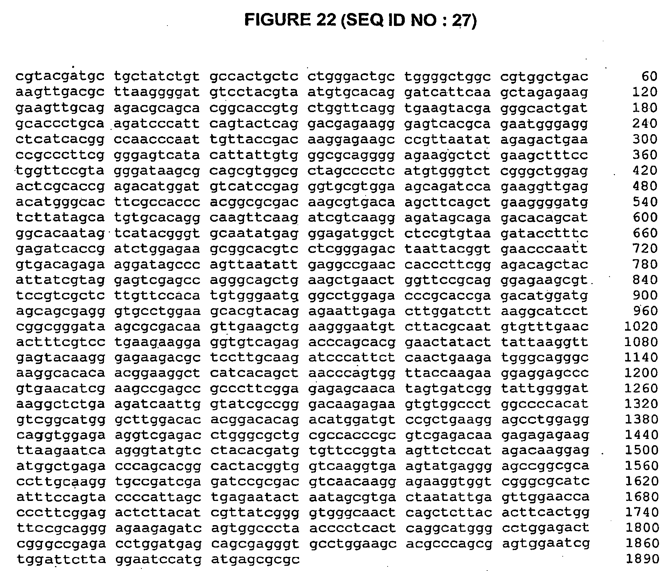

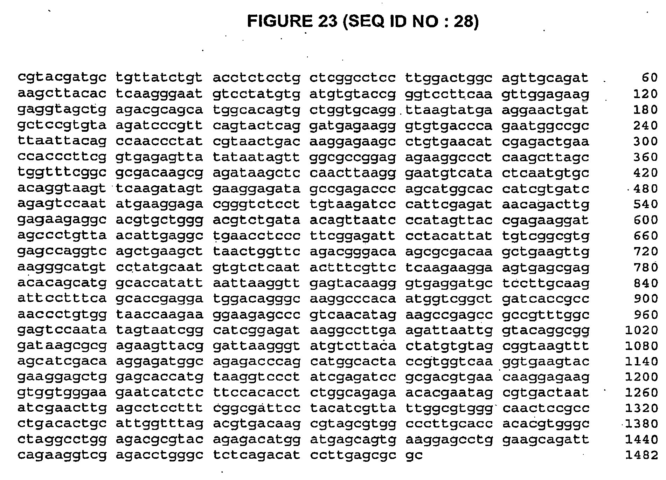

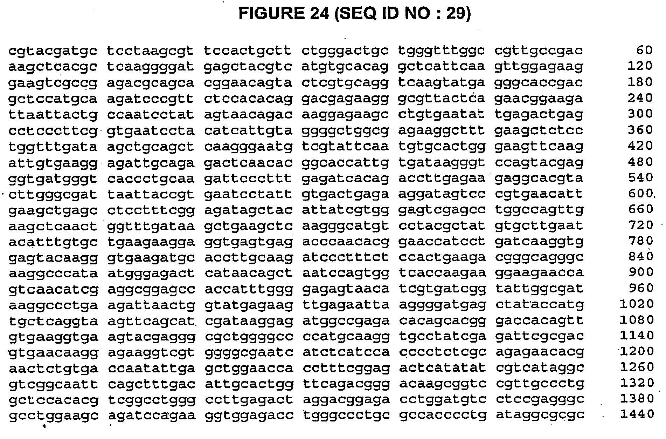

Par la suite, ces polypeptides chimériques.optimisés pour une expression en mammifère ont été insérés dans le génome de la souche virale atténuée Schwarz du virus de la rougeole (MVSchw). Les figures 15 à 29 montrent les séquences en acides aminés de cinq polypeptides chimériques préférés et les polynucléotides les codant.

Ces polypeptides chimériques ont été construits selon les conditions d'optimisation suivantes :
informations d'optimisation de ces séquences
   - (No Cai=0.806, %GC moyen = 53.46, distribution en GC : homogène environ 50%) ;
   - élimination des séquences "TATA box" interne, portion riche en AT ou GC, les éléments de séquences ARE, INS et CRS, séquences répétées, séquences de structure d'ARN secondaire, séquences d'épissage cryptique ;
   - élimination des sites de restriction suivants : Nhel, BamHI, XhoI, EcoRI, KpnI, SaII, BspEI, BgIII, NotI, BssHII, BsiWI, à l'exception pour BsiWI en première position et BssHII en dernière position ;
   - élimination de motifs TTTT, TTTAA, AAAGGG, AAAAGG, GGGAAA, GGGGAA et leurs complémentaires TTCCCC, TTTCCC, CCTTTT, CCCTTT.

**TABLEAU 1: Réponse d'anticorps dirigée contre le virus MVSchw chez les souris CD46⁺/IFNAR inoculées i.p. par MV_{Schw}-[EDIII]_{DV1}**

| Immunisation (mois) | MVAq titre^{c} | DV-1 Ag titre^{d} | DV-1 REDIII titre^{e} | Anti-DV-1 FRNT75^{f} |
|---|---|---|---|---|
| 1^{a} | 15,000 | < 100 | < 100 | ND |
| 2^{b} | 40,000 | 100 | 10 | <10 |

| | | | | |
|---|---|---|---|---|
| a. 10⁴ TCIP50 de MV-DV1[EDIII+M¹⁻⁴⁰] ont été donnés i.p. à quatre souris adultes b. Les individus ont eu une injection de rappel avec 10⁴ TCIP50 de MV-DV1[EDIII+M¹⁻⁴⁰] c. Déterminé par ELISA (Trinity Biotech) sur des sérums regroupés (pooled) et inactivés par la chaleur. d. Déterminé par ELISA sur des sérums regroupés (pooled) et inactivés par la chaleur. Des plaques de microtitrage ont été recouvertes de 5 x 10⁵ FFU de sucrose purifié, FGA/NA d1d comme antigène viral. e. Déterminé par ELISA sur des sérums regroupés (pooled) et inactivés par la chaleur. Des plaques de microtitrage plaques ont été recouvertes de 50 ng de EDIII recombinant hautement purifié du DV1 comme antigène viral. f. Les anticorps de neutralisation anti-DV1 ont été détectés par l'utilisation du test de séro-neutralisation par réduction des plages (ou "Focus Reduction Neutralization Test" (FRNT)). Des sérums regroupés (pooled) et inactivés par la chaleur ont été incubés avec la lignée Hawaï DV1 et le titrage de virus a été effectué sur des cellules Vero par le test d'immunodétection des plages. FRNT75, la dilution de sérum la plus haute testée qui a réduit le nombre de FFU d'au moins 75%. | | | | |

**TABLEAU 2: Réponse d'anticorps dirigée contre le virus MVSchw chez les souris CD46⁺/IFNAR inoculées i.p. par MV_{Schw}-[EDIII+M¹⁻⁴⁰]_{DV1}**

| Immunisation (mois) | MV Ag titre^{e} | DV-1 Ag titre^{f} | DV-1 rEDIII titre^{g} | Anti-DV-1 FRNT75^{h} |
|---|---|---|---|---|
| 1^{a} | 15,000 | < 100 | ≤ 100 | ND |
| 2^{b} | 40,000 | 1,600 | 400 | 10 |
| 3 | 30,000 | 1,000 | 600 | 10 |
| 6 | 20,000 | 500 | 100 | 40 |
| 7^{c} | 20,000 | 20,000 | 100,000 | 800 |
| 9 | 10,000 | 2,000 | 10,000 | 100 |
| 10^{d} | 10,000 | 200,000 | 800,000 | 4,000 |

| | | | | |
|---|---|---|---|---|
| a. 10⁴ TCIP50 de MV-DV1[EDIII+M¹⁻⁴⁰] ont été donnés i.p. à quatre souris adultes b. Les individus ont eu une injection de rappel avec 10⁴ TCIP50 de MV-DV1[EDIII+M¹⁻⁴⁰] c. Les souris immunisées ont eu une injection de rappel de 5 µg de protéines secrétées totales provenant du surnageant de cellules S2 exprimant rDV1[EDIII+M¹⁻⁴⁰] en présence de l'adjuvant Alugel. d. Les souris immunisées ont été inoculées i.p. avec 10⁷ FFU de FGA/NA d1d de la lignée DV1 pour trois semaines. e. Déterminé par ELISA (Trinity Biotech) sur des sérums regroupés (pooled) et inactivés par la chaleur. f. Déterminé par ELISA sur des sérums regroupés (pooled) et inactivés par la chaleur. Des plaques de microtitrage ont été recouvertes de 5 x 10⁵ FFU de sucrose purifié, FGA/NA d1d comme antigène viral. g. Déterminé par ELISA sur des sérums regroupés (pooled) et inactivés par la chaleur. Des plaques de microtitrage ont été recouvertes de 50 ng de EDIII recombinant hautement purifié du DV1 comme antigène viral. h. Les anticorps de neutralisation anti-DV1 ont été détectés par l'utilisation du FNRT. Des sérums regroupés (pooled) et inactivés par la chaleur ont été incubés avec la lignée Hawaï DV1 et le titrage de virus a été effectué sur des cellules Vero par l'utilisation du test d'immunodétection des plages. FRNT75, la dilution de sérum la plus haute testée qui a réduit le nombre de FFU d'au moins 75%. | | | | |

## Revendications

1. Polypeptide chimérique consistant en un peptide de sous-domaine de la protéine E de *Flaviviridae* lié à un peptide de sous-domaine de la protéine de membrane M de *Flaviviridae* et, le cas échéant, un segment de liaison liant le peptide de sous-domaine de la protéine E au peptide de sous-domaine de la protéine M, dans lequel le peptide de sous-domaine de la protéine M consiste en:
- l'ectodomaine 1-40 comprenant une séquence en acides aminés allant de la position 123 à 162 de la SEQ ID NO : 3; ou
- la séquence apoptoM comprenant une séquence en acides aminés allant de la position 154 à 162 de la SEQ ID NO : 3 ou allant de la position 122 à 132 de la SEQ ID NO :12.

2. Le polypeptide chimérique selon la revendication 1, **caractérisé en ce que** le peptide de sous-domaine de la protéine E consiste en l'ectodomaine III comprenant une séquence en acides aminés telle que définie dans l'une quelconque des séquences suivantes :
- acides aminés 18 à 120 de la SEQ ID NO : 20;
- acides aminés 171 à 273 de la SEQ ID NO : 20;
- acides aminés 324 à 426 de la SEQ ID NO : 20;
- acides aminés 477 à 579 de la SEQ ID NO : 20

3. Le polypeptide chimérique selon la revendication 1, **caractérisé en ce que** le peptide de sous-domaine de la protéine E consiste en un tétramère de l'ectodomaine III des virus de la Dengue 1, 2, 3 et 4 comprenant une séquence allant de l'acide aminé 18 à 429 de la SEQ ID NO :24.

4. Le polypeptide chimérique selon l'une quelconque des revendications 1 à 3, comprenant un segment de liaison liant le peptide de sous-domaine de la protéine E au peptide de sous-domaine de la protéine M.

5. Le polypeptide selon la revendication 4, **caractérisé en ce que** le segment de liaison est un pentapeptide ayant pour séquence : RRDKR ou RREKR.

6. Le polypeptide chimérique selon l'une quelconque des revendications 1 à 5, comprenant une séquence en acides aminés telle que définie dans l'une quelconque des séquences suivantes :
- acides aminés 18 à 624 de la SEQ ID NO : 20;
- acides aminés 18 à 609 de la SEQ ID NO : 21;
- acides aminés 18 à 624 de la SEQ ID NO : 22;
- acides aminés 18 à 489 de la SEQ ID NO : 23;et
- acides aminés 21 à 474 de la SEQ ID NO : 24.

7. Le polypeptide chimérique selon l'une quelconque des revendications 1 à 6, **caractérisé en ce que** le *Flaviviridae* est sélectionné dans le groupe constitué par les virus du Nil Occidental, de la Dengue, de l'encéphalite japonaise et de la fièvre jaune.

8. Un polynucléotide isolé ou purifié codant pour un polypeptide chimérique tel que défini dans l'une quelconque des revendications 1 à 7.

9. Le polynucléotide selon la revendication 8, **caractérisé en ce qu'**il comprend une séquence nucléotidique telle que définie dans l'une quelconque des séquences suivantes :
- SEQ ID NOS : 25 à 29.

10. Un vecteur viral recombinant de la rougeole dans le génome duquel est inséré un polynucléotide selon la revendication 8 ou9.

11. Le vecteur viral recombinant selon la revendication10, **caractérisé en ce qu'**il s'agit d'un vecteur viral vivant rougeole souche Schwarz.

12. Le vecteur viral recombinant selon la revendication 10 sélectionné dans le groupe de vecteurs viraux constitués par ceux déposés à la CNCM sous les numéros I-3440, I-3442, I-3452, I-3453, I-3454, I-3455, I-3619, I-3620, I-3621, I-3622 et I-3623.

13. Utilisation d'un vecteur viral selon l'une quelconque des revendications 10 à 12 pour la préparation d'une composition immunogène destinée à la prévention ou au traitement d'une infection à *Flaviviridae* chez une espèce sensible.

14. Vecteur de clonage ou d'expression comprenant un polynucléotide défini à la revendication8.

15. Composition immunogène destinée à la prévention et/ou au traitement d'une infection à *Flaviviridae* chez une espèce sensible, **caractérisée en ce qu'**elle comprend au moins un des éléments suivants :
- un polypeptide chimérique tel que défini dans l'une quelconque des revendications 1 à7;
- un polynucléotide selon la revendication8;
- un vecteur viral recombinant selon la revendication 10;
- un vecteur de clonage et/ou d'expression selon la revendication 14.

16. Composition immunogène selon la revendication 15, **caractérisée en ce qu'**elle comprend un véhicule pharmaceutiquement acceptable.

17. Utilisation selon la revendication 13, **caractérisée en ce que** le *Flaviviridae* est sélectionné dans le groupe constitué par le virus de la dengue, le virus de la fièvre jaune, le virus de l'encéphalite japonaise et le virus de la fièvre du Nil Occidental.

## Patentansprüche

1. Chimäres Polypeptid, bestehend aus einem Peptid des Unterbereichs des Proteins E von Flaviviridae, gebunden an ein Peptid des Unterbereichs des Membranproteins M von Flaviviridae und, gegebenenfalls, ein Verbindungssegment, welches das Peptid des Unterbereichs des Proteins E an das Peptid des Unterbereichs des Proteins M bindet, worin das Peptid des Unterbereichs des Proteins M aus:
- dem 1-40 Ekto-Bereich, umfassend eine Aminosäuresequenz, welche von der Position 123 bis 162 der SEQ ID Nr: 3 reicht oder
- der ApoptoM-Sequenz, umfassend eine Aminosäuresequenz, welche von der Position 154 bis 162 der SEQ ID Nr: 3 reicht oder von der Position 122 bis 132 der SEQ ID Nr: 12
besteht.

2. Chimäres Polypeptid gemäß Anspruch 1, **dadurch gekennzeichnet, dass** das Peptid des Unterbereichs des Proteins E aus dem Ekto-Bereich III besteht, umfassend eine Aminosäuresequenz, wie sie in einer der nachfolgenden Sequenzen definiert ist:
- Aminosäuren 18 bis 120 der SEQ ID Nr: 20;
- Aminosäuren 171 bis 273 der SEQ ID Nr: 20;
- Aminosäuren 324 bis 426 der SEQ ID Nr: 20;
- Aminosäuren 477 bis 579 der SEQ ID Nr: 20.

3. Chimäres Polypeptid gemäß Anspruch 1, **dadurch gekennzeichnet, dass** das Peptid des Unterbereichs des Proteins E aus einem Tetramer des Ekto-Bereichs III des Dengue-Virus 1, 2, 3 und 4 besteht, umfassend eine Sequenz, welche von der Aminosäure 18 bis 429 der SEQ ID Nr: 24 reicht.

4. Chimäres Polypeptid gemäß einem jeden der Ansprüche 1 bis 3, umfassend ein Verbindungssegment, welches das Peptid des Unterbereichs des Proteins E an das Peptid des Unterbereichs des Proteins M bindet.

5. Polypeptid gemäß Anspruch 4, **dadurch gekennzeichnet, dass** das Verbindungssegment ein Pentapeptid ist, das die Sequenz RRDKR oder RREKR aufweist.

6. Chimäres Polypeptid gemäß einem jeden der Ansprüche 1 bis 5, umfassend eine Aminosäuresequenz, wie sie in einer der nachfolgenden Sequenzen definiert ist.
- Aminosäuren 18 bis 624 von SEQ ID Nr: 20;
- Aminosäuren 18 bis 609 von SEQ ID Nr: 21;
- Aminosäuren 18 bis 624 von SEQ ID Nr: 22;
- Aminosäuren 18 bis 489 von SEQ ID Nr: 23; und
- Aminosäuren 21 bis 474 von SEQ ID Nr: 24.

7. Chimäres Polypeptid gemäß einem jeden der Ansprüche 1 bis 6, **dadurch gekennzeichnet, dass** Flaviviridae aus der Gruppe ausgewählt ist, bestehend aus den West-Nil-, Dengue-, Japan Enzephalitis- und Gelbfieber-Viren.

8. Isoliertes oder gereinigtes Polynukleotid, das für ein chimäres Polypeptid, wie es in einem der Ansprüche 1 bis 7 definiert ist, kodiert.

9. Polynukleotid gemäß Anspruch 8, **dadurch gekennzeichnet, dass** es eine Nukleotidsequenz umfasst, wie sie in einem jeden der nachfolgenden Sequenzen definiert ist:
- SEQ ID Nrn: 25 bis 29.

10. Rekombinanter viraler Vektor von Masern in dem Genom, in das ein Polypeptid gemäß Anspruch 8 oder 9 inseriert ist.

11. Rekombinanter viraler Vektor gemäß Anspruch 10, **dadurch gekennzeichnet, dass** es sich um einen lebenden viralen Vektor von Masern des Stammes Schwarz handelt.

12. Rekombinanter viraler Vektor gemäß Anspruch 10 ausgewählt aus der Gruppe der viralen Vektoren, bestehend aus jenen, die bei CNCM unter den Nummern 1-3440, 1-3442, 1-3452, 1-3453, 1-3454, 1-3455, 1-3619, 1-3620, 1-3621, 1-3622 und 1-3623 hinterlegt sind.

13. Verwendung eines viralen Vektors gemäß einem jeden der Ansprüche 10 bis 12 zur Herstellung einer immunogenen Zusammensetzung zur Vorbeugung oder Behandlung einer Flaviviridae-Infektion bei einer empfindlichen Spezies.

14. Klonierungs- oder Exprimierungsvektor, umfassend ein Polynukleotid, das in Anspruch 8 definiert ist.

15. Immunogene Zusammensetzung zur Vorbeugung und/oder Behandlung einer Infektion mit Flaviviridae bei einer sensiblen Spezies, **dadurch gekennzeichnet, dass** sie wenigstens eines der folgenden Elemente umfasst:
- ein chimäres Polypeptid, wie es in einem jeden der Ansprüche 1 bis 7 definiert ist;
- ein Polynukleotid gemäß Anspruch 8;
- einen rekombinanten viralen Vektor gemäß Anspruch 10;
- einen Klonierungs- und/oder Exprimierungsvektor gemäß Anspruch 14.

16. Immunogene Zusammensetzung gemäß Anspruch 15, **dadurch gekennzeichnet, dass** sie einen pharmazeutisch annehmbaren Träger umfasst.

17. Verwendung gemäß Anspruch 13, **dadurch gekennzeichnet, dass** das Flaviviridae aus der Gruppe ausgewählt ist, bestehend aus dem Dengue-Virus, dem Gelbfieber-Virus, dem Japan Enzephalitis-Virus und dem West-Nil-Virus.

## Claims

1. A chimeric polypeptide consisting of a peptide of a subdomain of the E protein of *Flaviviridae* bound to a peptide of a subdomain of the membrane M protein of *Flaviviridae* and, if appropriate, a binding segment binding the peptide of a subdomain of the E protein to the peptide of a subdomain of the M protein, in which the peptide of a subdomain of the M protein consists of:
• the ectodomain 1-40 comprising an amino acid sequence ranging from position 123 to position 162 of SEQ ID NO: 3; or
• the apoptoM sequence comprising an amino acid sequence ranging from position 154 to 162 of SEQ ID NO: 3 or ranging from position 122 to 132 of SEQ ID NO: 12.

2. The chimeric polypeptide according to claim 1, **characterized in that** the peptide of a subdomain of the E protein consists of the ectodomain III comprising an amino acid sequence as defined in any one of the following sequences:
• amino acids 18 to 120 of SEQ ID NO: 20;
• amino acids 171 to 273 of SEQ ID NO: 20;
• amino acids 324 to 426 of SEQ ID NO: 20;
• amino acids 477 to 579 of SEQ ID NO: 20.

3. The chimeric polypeptide according to claim 1, **characterized in that** the peptide of a subdomain of the E protein consists of a tetramer of the ectodomain III of the dengue 1, 2, 3 or 4 virus comprising a sequence ranging from amino acid 18 to 429 of SEQ ID NO: 24.

4. The chimeric polypeptide according to any one of claims 1 to 3, further comprising a binding segment binding the peptide of a subdomain of the E protein to the peptide of a subdomain of the M protein.

5. The polypeptide according to claim 4, **characterized in that** the binding segment is a pentapeptide with sequence: RRDKR or RREKR.

6. The chimeric polypeptide according to any one of claims 1 to 5, comprising an amino acid sequence as defined in any one of the following sequences:
• amino acids 18 to 624 of SEQ ID NO: 20;
• amino acids 18 to 609 of SEQ ID NO: 21;
• amino acids 18 to 624 of SEQ ID NO: 22;
• amino acids 18 to 489 of SEQ ID NO: 23; and
• amino acids 21 to 474 of SEQ ID NO: 24.

7. The chimeric polypeptide according to any one of claims 1 to 6, **characterized in that** the *Flaviviridae* is selected from the group constituted by West Nile virus, dengue virus, Japanese encephalitis virus and yellow fever virus.

8. An isolated or purified polynucleotide coding for a chimeric polypeptide as defined in any one of claims 1 to 7.

9. The polynucleotide according to claim 8, **characterized in that** it comprises a nucleotide sequence as defined in any one of the following sequences:
• SEQ ID NOs: 25 to 29.

10. A recombinant measles viral vector with a polynucleotide according to claim 8 or claim 9 inserted into the genome thereof.

11. The recombinant viral vector according to claim 10, **characterized in that** it is a live measles, Schwarz strain, viral vector.

12. The recombinant viral vector according to claim 10, selected from the group of viral vectors constituted by those deposited at the CNCM with accession numbers 1-3440, I-3442, I-3452, I-3453, I-3454, I-3455, I-3619, I-3620, I-3621, I-3622 and 1-3623.

13. Use of a viral vector according to any one of claims 10 to 12, for the preparation of an immunogenic composition intended for the prevention or treatment of a *Flaviviridae* infection in a sensitive species.

14. A cloning or expression vector comprising a polynucleotide as defined in claim 8.

15. An immunogenic composition intended for the prevention and/or treatment of a *Flaviviridae* infection in a sensitive species, **characterized in that** it comprises at least one of the following elements:
• a chimeric polypeptide as defined in any one of claims 1 to 7;
• a polynucleotide according to claim 8;
• a recombinant viral vector according to claim 10;
• a cloning and/or expression vector according to claim 14.

16. The immunogenic composition according to claim 15, **characterized in that** it comprises a pharmaceutically acceptable vehicle.

17. Use according to claim 13, **characterized in that** the *Flaviviridae* is selected from the group constituted by the dengue virus, yellow fever virus, Japanese encephalitis virus and West Nile fever virus.


### SEQUENCE LISTING

<110> INSTITUT PASTEUR CENTRE NATIONAL DE LA RECHERCHE SCIENTIFIQUE
<120> POLYPEPTIDES CHIMERIQUES ET LEURS APPLICATIONS THERAPEUTIQUES CONTRE UNE INFECTION A FLAVIVIRIDAE
<130> B6802A
<150> CA 2508266 <151> 2005-06-20
<160> 33
<170> PatentIn version 3.3
<210> 1
   <211> 119
   <212> PRT
   <213> Artificial
<220>
   <223> fusion sequence comprising domain(s) of Flaviviridae strains.
<400> 1
   Met Leu Leu Ser Val Pro Leu Leu Leu Gly Leu Leu Gly Leu Ala Val
   1               5                   10                  15
   Ala Arg Ser Lys Gly Met Ser Tyr Val Met Cys Thr Gly Ser Phe Lys
               20                  25                  30
   Leu Glu Lys Glu Val Ala Glu Thr Gln His Gly Thr Val Leu Val Gln
           35                  40                  45
   Val Lys Tyr Glu Gly Thr Asp Ala Pro Cys Lys Ile Pro Phe Ser Thr
       50                  55                  60
   Gln Asp Glu Lys Gly Val Thr Gln Asn Gly Arg Leu Ile Thr Ala Asn
   65                  70                  75                  80
   Pro Ile Val Thr Asp Lys Glu Lys Pro Val Asn Ile Glu Thr Glu Pro
                   85                  90                  95
   Pro Phe Gly Glu Ser Tyr Ile Ile Val Gly Ala Gly Glu Lys Ala Leu
               100                 105                 110
   Lys Leu Ser Trp Phe Lys Arg
           115
<210> 2
   <211> 384
   <212> DNA
   <213> Artificial
<220>
   <223> fusion sequence comprising domain(s) of Flaviviridae strains.
<400> 2
   cgtacgatgc tgctatccgt gccgttgctg ctcggcctcc tcggcctggc cgtcgccaga    60
   tctaaaggga tgtcatatgt gatgtgcaca ggctcattta agctagagaa ggaagtggct   120
   gagacccagc atgggactgt cctagtgcag gttaaatacg aaggaacaga tgcgccatgc   180
   aagatcccct tttcgaccca agatgagaaa ggagtgaccc agaatgggag attgataaca   240
   gccaatccca tagttactga caaagaaaaa ccagtcaaca ttgagacaga accacctttt   300
   ggtgagagct acatcatagt aggggcaggt gaaaaagctt tgaaactaag ctggttcaag   360
   cgatagcggc cgctataagc gcgc                                          384
<210> 3
   <211> 162
   <212> PRT
   <213> Artificiel
<220>
   <223> fusion sequence comprising domain(s) of Flaviviridae strains.
<400> 3
   Met Leu Leu Ser Val Pro Leu Leu Leu Gly Leu Leu Gly Leu Ala Val
   1               5                   10                  15
   Ala Arg Ser Lys Gly Met Ser Tyr Val Met Cys Thr Gly Ser Phe Lys
               20                  25                  30
   Leu Glu Lys Glu Val Ala Glu Thr Gln His Gly Thr Val Leu Val Gln
           35                  40                  45
   Val Lys Tyr Glu Gly Thr Asp Ala Pro Cys Lys Ile Pro Phe Ser Thr
       50                  55                  60
   Gln Asp Glu Lys Gly Val Thr Gln Asn Gly Arg Leu Ile Thr Ala Asn
   65                  70                  75                  80
   Pro Ile Val Thr Asp Lys Glu Lys Pro Val Asn Ile Glu Thr Glu Pro
                   85                  90                  95
   Pro Phe Gly Glu Ser Tyr Ile Ile Val Gly Ala Gly Glu Lys Ala Leu
               100                 105                 110
   Lys Leu Ser Trp Phe Arg Arg Asp Lys Arg Ser Val Ala Leu Ala Pro
           115                 120                 125
   His Val Gly Leu Gly Leu Glu Thr Arg Thr Glu Thr Trp Met Ser Ser
       130                 135                 140
   Glu Gly Ala Trp Lys Gln Ile Gln Lys Val Glu Thr Trp Ala Leu Arg
   145                 150                 155                 160
   His Pro
<210> 4
   <211> 516
   <212> DNA
   <213> Artificial
<220>
   <223> fusion sequence comprising domain(s) of Fiaviviridae strains.
<400> 4
   cgtacgatgc tgctatccgt gccgttgctg ctcggcctcc tcggcctggc cgtcgccaga    60
   tctaaaggga tgtcatatgt gatgtgcaca ggctcattta agctagagaa ggaagtggct   120
   gagacccagc atgggactgt cctagtgcag gttaaatacg aaggaacaga tgcgccatgc   180
   aagatcccct tttcgaccca agatgagaaa ggagtgaccc agaatgggag attgataaca   240
   gccaatccca tagttactga caaagaaaaa ccagtcaaca ttgagacaga accacctttt   300
   ggtgagagct acatcatagt aggggcaggt gaaaaagctt tgaaactaag ctggttccga   360
   cgagacaaac gttccgtggc actggcccca cacgtgggac ttggtctaga aacaagaacc   420
   gaaacatgga tgtcctctga aggcgcctgg aaacaaatac aaaaagtgga gacttgggct   480
   ttgagacacc catgatagcg gccgctataa gcgcgc                             516
<210> 5
   <211> 516
   <212> DNA
   <213> Artificial
<220>
   <223> fusion sequence comprising domain(s) of Flaviviridae strains.
<400> 5
   cgtacgatgc tgctatccgt gccgttgctg ctcggcctcc tcggcctggc cgtcgccaga    60
   tctaaaggga tgtcatatgt gatgtgcaca ggctcattta agctagagaa ggaagtggct   120
   gagacccagc atgggactgt cctagtgcag gttaaatacg aaggaacaga tgcgccatgc   180
   aagatcccct tttcgaccca agatgagaaa ggagtgaccc agaatgggag attgataaca   240
   gccaatccca tagttactga caaagaaaaa ccagtcaaca ttgagacaga accacctttt   300
   ggtgagagct acatcatagt aggggcaggt gaaaaagctt tgaaactaag ctggttccga   360
   cgagacaaac gttccgtggc actggcccca cacgtgggac ttggtctaga aacaagaacc   420
   gaaacatgga tgtcctctga aggcgcctgg aaacaaatac aaaaagtgga gacttgggct   480
   ttgagacacc catgatagcg gccgctataa gcgcgc                             516
<210> 6
   <211> 173
   <212> PRT
   <213> Artificial
<220>
   <223> fusion sequence comprising domain(s) of Flaviviridae strains.
<400> 6
   Arg Thr Met Leu Leu Ser Val Pro Leu Leu Leu Gly Leu Leu Gly Leu
   1               5                   10                  15
   Ala Val Ala Arg Ser Lys Gly Thr Thr Tyr Gly Met Cys Thr Glu Lys
               20                  25                  30
   Phe Ser Phe Ala Lys Asn Pro Ala Asp Thr Gly His Gly Thr Val Val
           35                  40                  45
   Ile Glu Leu Ser Tyr Ser Gly Ser Asp Gly Pro Cys Lys Ile Pro Ile
       50                  55                  60
   Val Ser Val Ala Ser Leu Asn Asp Met Thr Pro Val Gly Arg Leu Val
   65                  70                  75                  80
   Thr Val Asn Pro Phe Val Ala Thr Ser Ser Ala Asn Ser Lys Val Leu
                   85                  90                  95
   Val Glu Met Glu Pro Pro Phe Gly Asp Ser Tyr Ile Val Val Gly Arg
               100                 105                 110
   Gly Asp Lys Gln Ile Asn His His Trp His Lys Lys Arg Ser Arg Arg
           115                 120                 125
   Ser Val Ser Val Gln Thr His Gly Glu Ser Ser Leu Val Asn Lys Lys
       130                 135                 140
   Glu Ala Trp Leu Asp Ser Thr Lys Ala Thr Arg Tyr Leu Met Lys Thr
   145                 150                 155                 160
   Glu Asn Trp Ile Val Arg Asn Pro Arg Pro Leu Ala Arg
                   165                 170
<210> 7
   <211> 528
   <212> DNA
   <213> Artificial
<220>
   <223> fusion sequence comprising domain(s) of Flaviviridae strains.
<400> 7
   cgtacgatgc tgctatccgt gccgttgctg ctcggcctcc tcggcctggc cgtcgccaga 60
   tctaaaggca caacctatgg catgtgcaca gaaaaattct cgttcgcgaa aaatccggcg 120
   gacactggtc acggaacagt tgtcattgaa ctttcctact ctgggagtga tggcccttgc 180
   aaaattccga ttgtctccgt tgcgagcctc aatgacatga cccccgtcgg gcggctggtg 240
   acagtgaacc ccttcgtcgc gacttccagc gccaactcaa aggtgctagt cgagatggaa 300
   ccccccttcg gagactccta catcgtagtt ggaaggggag acaagcagat caaccaccat 360
   tggcacaaaa agcgaagcag gagatccgtg tcggtccaaa cacatgggga gagttcacta 420
   gtgaataaaa aagaggcttg gctggattca acgaaagcca cacgatacct catgaaaact 480
   gagaactgga tcgtaaggaa tccttgatag cggccgctat aagcgcgc 528
<210> 8
   <211> 264
   <212> PRT
   <213> Artificial
<220>
   <223> fusion sequence comprising domain(s) of Flaviviridae strains.
<400> 8
   Arg Thr Met Leu Leu Ser Val Pro Leu Leu Leu Gly Leu Leu Gly Leu
   1               5                   10                  15
   Ala Val Ala Arg Ser Lys Gly Met Ser Tyr Val Met Cys Thr Gly Ser
               20                  25                  30
   Phe Lys Leu Glu Lys Glu Val Ala Glu Thr Gln His Gly Thr Val Leu
           35                  40                  45
   Val Gln Val Lys Tyr Glu Gly Thr Asp Ala Pro Cys Lys Ile Pro Phe
       50                  55                  60
   Ser Thr Gln Asp Glu Lys Gly Val Thr Gln Asn Gly Arg Leu Ile Thr
   65                  70                  75                  80
   Ala Asn Pro Ile Val Thr Asp Lys Glu Lys Pro Val Asn Ile Glu Thr
                   85                  90                  95
   Glu Pro Pro Phe Gly Glu Ser Tyr Ile Ile Val Gly Ala Gly Glu Lys
               100                 105                 110
   Ala Leu Lys Leu Ser Trp Phe Lys Lys Gly Ser Ser Ile Gly Lys Met
           115                 120                 125
   Phe Glu Ala Thr Ala Gly Gly Ser Gly Gly Lys Gly Met Ser Tyr Ser
       130                 135                 140
   Met Cys Thr Gly Lys Phe Lys Ile Val Lys Glu Ile Ala Glu Thr Gln
   145                 150                 155                 160
   His Gly Thr Ile Val Ile Arg Val Gln Tyr Glu Gly Asp Gly Ser Pro
                   165                 170                 175
   Cys Lys Ile Pro Phe Glu Ile Met Asp Leu Glu Lys Arg His Val Leu
               180                 185                 190
   Gly Arg Leu Ile Thr Val Asn Pro Ile Val Thr Glu Lys Asp Ser Pro
           195                 200                 205
   Val Asn Ile Glu Ala Glu Pro Pro Phe Gly Asp Ser Tyr Ile Ile Ile
       210                 215                 220
   Gly Val Glu Pro Gly Gln Leu Lys Leu Asn Trp Phe Lys Lys Gly Ser
   225                 230                 235                 240
   Ser Ile Gly Gln Met Phe Glu Thr Thr Met Ser Gly Arg Val Glu Thr
                   245                 250                 255
   Trp Ala Leu Arg His Pro Ala Arg
               260
<210> 9
   <211> 798
   <212> DNA
   <213> Artificial
<220>
   <223> fusion sequence comprising domain(s) of Flaviviridae strains.
<400> 9
   cgtacgatgc tgctatccgt gccgttgctg ctcggcctcc tcggcctggc cgtcgccaga      60
   tctaaaggga tgtcatatgt gatgtgcaca ggctcattta agctagagaa ggaagtggct     120
   gagacccagc atgggactgt cctagtgcag gttaaatacg aaggaacaga tgcgccatgc     180
   aagatcccct tttcgaccca agatgagaaa ggagtgaccc agaatgggag attgataaca     240
   gccaatccca tagttactga caaagaaaaa ccagtcaaca ttgagacaga accacctttt     300
   ggtgagagct acatcatagt aggggcaggt gaaaaagctt tgaaactaag ctggttcaag     360
   aaaggaagca gcatagggaa aatgttcgaa gcaaccgccg gaggatcagg agggaaagga     420
   atgtcatact ctatgtgtac aggaaagttt aaaattgtga aggaaatagc agaaacacaa     480
   catggaacaa tagttatcag agtacaatat gaaggggacg gctctccatg taagatccct     540
   tttgagataa tggatttgga aaaaagacac gtcttaggtc gcctgattac agttaacccg     600
   atcgtaacag aaaaagatag cccagtcaac atagaagcag aacctccatt cggagacagc     660
   tacatcatca taggagtaga gccgggacaa ttgaaactca actggtttaa gaaaggaagt     720
   tccatcggcc aaatgtttga gacaacaatg agcggccgcg tggagacttg ggctttgaga     780
   cacccatagt aagcgcgc                                                   798
<210> 10
   <211> 264
   <212> PRT
   <213> Artificial
<220>
   <223> fusion sequence comprising domain(s) of Flaviviridae strains.
<400> 10
   Arg Thr Met Leu Leu Ser Val Pro Leu Leu Leu Gly Leu Leu Gly Leu
   1               5                   10                  15
   Ala Val Ala Arg Ser Lys Gly Met Ser Tyr Ala Met Cys Leu Asn Thr
               20                  25                  30
   Phe Val Leu Lys Lys Glu Val Ser Glu Thr Gln His Gly Thr Ile Leu
   35 40 45
   Ile Lys Val Glu Tyr Lys Gly Glu Asp Ala Pro Cys Lys Ile Pro Phe
   50 55 60
   Ser Thr Glu Asp Gly Gln Gly Lys Ala His Asn Gly Arg Leu Ile Thr
   65 70 75 80
   Ala Asn Pro Val Val Thr Lys Lys Glu Glu Pro Val Asn Ile Glu Ala
   85 90 95
   Glu Pro Pro Phe Gly Glu Ser Asn Ile Val Ile Gly Ile Gly Asp Lys
   100 105 110
   Ala Leu Lys Ile Asn Trp Tyr Lys Lys Gly Ser Ser Ile Gly Lys Met
   115 120 125
   Phe Glu Ala Thr Ala Gly Gly Ser Gly Gly Lys Gly Met Ser Tyr Thr
   130 135 140
   Met Cys Ser Gly Lys Phe Ser Ile Asp Lys Glu Met Ala Glu Thr Gln
   145 150 155 160
   His Gly Thr Thr Val Val Lys Val Lys Tyr Glu Gly Ala Gly Ala Pro
   165 170 175
   Cys Lys Val Pro Ile Glu Ile Arg Asp Val Asn Lys Glu Lys Val Val
   180 185 190
   Gly Arg Ile Ile Ser Ser Thr Pro Phe Ala Glu Asn Thr Asn Ser Val
   195 200 205
   Thr Asn Ile Glu Leu Glu Pro Pro Phe Gly Asp Ser Tyr Ile Val Ile
   210 215 220
   Gly Val Gly Asp Ser Ala Leu Thr Leu His Trp Phe Arg Lys Gly Ser
   225 230 235 240
   Ser Ile Gly Lys Met Phe Glu Ser Thr Tyr Ser Gly Arg Val Glu Thr
   245 250 255
   Trp Ala Leu Arg His Pro Ala Arg
   260
<210> 11
   <211> 798
   <212> DNA
   <213> Artificial
<220>
   <223> fusion sequence comprising domain(s) of Flaviviridae strains.
<400> 11
   cgtacgatgc tgctatccgt gccgttgctg ctcggcctcc tcggcctggc cgtcgccaga    60
   tctaagggga tgagctatgc aatgtgcttg aatacctttg tgttgaagaa agaagtctcc   120
   gaaacgcagc atgggacaat actcattaag gttgagtaca aaggggaaga tgcaccttgc   180
   aagattcctt tctccacaga ggatggacaa gggaaagctc acaatggtag actgatcaca   240
   gccaacccag tggtgaccaa gaaggaggag cctgtcaaca ttgaggctga acctcctttt   300
   ggggaaagta acatagtgat tggaattgga gacaaagcct tgaaaattaa ctggtacaag   360
   aagggaagct cgattgggaa gatgttcgag gccactgccg gtggttctgg tggtaaggga   420
   atgtcataca cgatgtgctc aggaaagttc tcaattgata aagagatggc agaaacacag   480
   catgggacaa cagtggtgaa agtcaagtat gagggtgctg gagctccatg taaagttccc   540
   atagagataa gagatgtgaa caaggaaaaa gtggttgggc gtatcatctc atctacccct   600
   tttgctgaga ataccaacag tgtgaccaat atagaattgg aacccccttt tggggatagc   660
   tacatagtaa taggtgtagg agacagtgca ttaacactcc attggttcag gaaagggagc   720
   tccattggca agatgtttga gtccacatac agcggccgcg tggagacttg ggctttgaga   780
   cacccatagt aagcgcgc                                                 798
<210> 12
   <211> 134
   <212> PRT
   <213> Artificial
<220>
   <223> fusion sequence comprising domain(s) of Flaviviridae strains.
<400> 12
   Arg Thr Met Leu Leu Ser Val Pro Leu Leu Leu Gly Leu Leu Gly Leu
   1               5                   10                  15
   Ala Val Ala Arg Ser Lys Gly Met Ser Tyr Val Met Cys Thr Gly Ser
               20                  25                  30
   Phe Lys Leu Glu Lys Glu Val Ala Glu Thr Gln His Gly Thr Val Leu
           35                  40                  45
   Val Gln Val Lys Tyr Glu Gly Thr Asp Ala Pro Cys Lys Ile Pro Phe
       50                  55                  60
   Ser Thr Gln Asp Glu Lys Gly Val Thr Gln Asn Gly Arg Leu Ile Thr
   65                  70                  75                  80
   Ala Asn Pro Ile Val Thr Asp Lys Glu Lys Pro Val Asn Ile Glu Thr
                   85                  90                  95
   Glu Pro Pro Phe Gly Glu Ser Tyr Ile Ile Val Gly Ala Gly Glu Lys
               100                 105                 110
   Ala Leu Lys Leu Ser Trp Phe Lys Arg Gly Arg Ile Glu Thr Trp Ile
   115 120 125
   Leu Arg His Pro Ala Arg
   130
<210> 13
   <211> 408
   <212> DNA
   <213> Artificial
<220>
   <223> fusion sequence comprising domain(s) of Flaviviridae strains.
<400> 13
   cgtacgatgc tgctatccgt gccgttgctg ctcggcctcc tcggcctggc cgtcgccaga 60
   tctaaaggga tgtcatatgt gatgtgcaca ggctcattta agctagagaa ggaagtggct 120
   gagacccagc atgggactgt cctagtgcag gttaaatacg aaggaacaga tgcgccatgc 180
   aagatcccct tttcgaccca agatgagaaa ggagtgaccc agaatgggag attgataaca 240
   gccaatccca tagttactga caaagaaaaa ccagtcaaca ttgagacaga accacctttt 300
   ggtgagagct acatcatagt aggggcaggt gaaaaagctt tgaaactaag ctggttcaag 360
   cgcggccgca ttgaaacctg gatcttgaga catccatagt aagcgcgc 408
<210> 14
   <211> 128
   <212> PRT
   <213> Flavivirus sp.
<400> 14
   Arg Thr Met Leu Leu Ser Val Pro Leu Leu Leu Gly Leu Leu Gly Leu
   1               5                   10                  15
   Ala Val Ala Arg Ser Lys Gly Thr Thr Tyr Gly Met Cys Thr Glu Lys
               20                  25                  30
   Phe Ser Phe Ala Lys Asn Pro Ala Asp Thr Gly His Gly Thr Val Val
           35                  40                  45
   Ile Glu Leu Ser Tyr Ser Gly Ser Asp Gly Pro Cys Lys Ile Pro Ile
       50                  55                  60
   Val Ser Val Ala Ser Leu Asn Asp Met Thr Pro Val Gly Arg Leu Val
   65                  70                  75                  80
   Thr Val Asn Pro Phe Val Ala Thr Ser Ser Ala Asn Ser Lys Val Leu
                   85                  90                  95
   Val Glu Met Glu Pro Pro Phe Gly Asp Ser Tyr Ile Val Val Gly Arg
               100                 105                 110
   Gly Asp Lys Gln Ile Asn His His Trp His Lys Arg Pro Leu Ala Arg
           115                 120                 125
<210> 15
   <211> 390
   <212> DNA
   <213> Flavivirus sp.
<400> 15
   cgtacgatgc tgctatccgt gccgttgctg ctcggcctcc tcggcctggc cgtcgccaga 60
   tctaaaggca caacctatgg catgtgcaca gaaaaattct cgttcgcgaa aaatccggcg 120
   gacactggtc acggaacagt tgtcattgaa ctttcctact ctgggagtga tggcccttgc 180
   aaaattccga ttgtctccgt tgcgagcctc aatgacatga cccccgtcgg gcggctggtg 240
   acagtgaacc ccttcgtcgc gacttccagc gccaactcaa aggtgctagt cgagatggaa 300
   ccccccttcg gagactccta catcgtagtt ggaaggggag acaagcagat caaccaccat 360
   tggcacaaat agcggccgct ataagcgcgc 390
<210> 16
   <211> 496
   <212> PRT
   <213> Artificial
<220>
   <223> fusion sequence comprising domain(s) of Flaviviridae strains.
<400> 16
   Arg Thr Met Leu Leu Ser Val Pro Leu Leu Leu Gly Leu Leu Gly Leu
   1               5                   10                  15
   Ala Val Ala Arg Ser Lys Gly Met Ser Tyr Val Met Cys Thr Gly Ser
               20                  25                  30
   Phe Lys Leu Glu Lys Glu Val Ala Glu Thr Gln His Gly Thr Val Leu
           35                  40                  45
   Val Gln Val Lys Tyr Glu Gly Thr Asp Ala Pro Cys Lys Ile Pro Phe
       50                  55                  60
   Ser Thr Gln Asp Glu Lys Gly Val Thr Gln Asn Gly Arg Leu Ile Thr
   65                  70                  75                  80
   Ala Asn Pro Ile Val Thr Asp Lys Glu Lys Pro Val Asn Ile Glu Thr
                   85                  90                  95
   Glu Pro Pro Phe Gly Glu Ser Tyr Ile Ile Val Gly Ala Gly Glu Lys
               100                 105                 110
   Ala Leu Lys Leu Ser Trp Phe Lys Lys Gly Ser Ser Ile Gly Lys Met
           115                 120                 125
   Phe Glu Ala Thr Ala Gly Gly Ser Gly Gly Lys Gly Met Ser Tyr Ser
   130 135 140
   Met Cys Thr Gly Lys Phe Lys Ile Val Lys Glu Ile Ala Glu Thr Gln
   145 150 155 160
   His Gly Thr Ile Val Ile Arg Val Gln Tyr Glu Gly Asp Gly Ser Pro
   165 170 175
   Cys Lys Ile Pro Phe Glu Ile Met Asp Leu Glu Lys Arg His Val Leu
   180 185 190
   Gly Arg Leu Ile Thr Val Asn Pro Ile Val Thr Glu Lys Asp Ser Pro
   195 200 205
   Val Asn Ile Glu Ala Glu Pro Pro Phe Gly Asp Ser Tyr Ile Ile Ile
   210 215 220
   Gly Val Glu Pro Gly Gln Leu Lys Leu Asn Trp Phe Lys Lys Gly Ser
   225 230 235 240
   Ser Ile Gly Gln Met Phe Glu Thr Thr Met Gly Gly Ser Lys Gly Met
   245 250 255
   Ser Tyr Ala Met Cys Leu Asn Thr Phe Val Leu Lys Lys Glu Val Ser
   260 265 270
   Glu Thr Gln His Gly Thr Ile Leu Ile Lys Val Glu Tyr Lys Gly Glu
   275 280 285
   Asp Ala Pro Cys Lys Ile Pro Phe Ser Thr Glu Asp Gly Gln Gly Lys
   290 295 300
   Ala His Asn Gly Arg Leu Ile Thr Ala Asn Pro Val Val Thr Lys Lys
   305 310 315 320
   Glu Glu Pro Val Asn Ile Glu Ala Glu Pro Pro Phe Gly Glu Ser Asn
   325 330 335
   Ile Val Ile Gly Ile Gly Asp Lys Ala Leu Lys Ile Asn Trp Tyr Lys
   340 345 350
   Lys Gly Ser Ser Ile Gly Lys Met Phe Glu Ala Thr Ala Gly Gly Ser
   355 360 365
   Gly Gly Lys Gly Met Ser Tyr Thr Met Cys Ser Gly Lys Phe Ser Ile
   370 375 380
   Asp Lys Glu Met Ala Glu Thr Gln His Gly Thr Thr Val Val Lys Val
   385 390 395 400
   Lys Tyr Glu Gly Ala Gly Ala Pro Cys Lys Val Pro Ile Glu Ile Arg
   405 410 415
   Asp Val Asn Lys Glu Lys Val Val Gly Arg Ile Ile Ser Ser Thr Pro
   420 425 430
   Phe Ala Glu Asn Thr Asn Ser Val Thr Asn Ile Glu Leu Glu Pro Pro
   435 440 445
   Phe Gly Asp Ser Tyr Ile Val Ile Gly Val Gly Asp Ser Ala Leu Thr
   450 455 460
   Leu His Trp Phe Arg Lys Gly Ser Ser Ile Gly Lys Met Phe Glu Ser
   465 470 475 480
   Thr Tyr Ser Gly Arg Val Glu Thr Trp Ala Leu Arg His Pro Ala Arg
   485 490 495
<210> 17
   <211> 1494
   <212> DNA
   <213> Artificial
<220>
   <223> fusion sequence comprising domain(s) of Flaviviridae strains.
<400> 17
   cgtacgatgc tgctatccgt gccgttgctg ctcggcctcc tcggcctggc cgtcgccaga     60
   tctaaaggga tgtcatatgt gatgtgcaca ggctcattta agctagagaa ggaagtggct    120
   gagacccagc atgggactgt cctagtgcag gttaaatacg aaggaacaga tgcgccatgc    180
   aagatcccct tttcgaccca agatgagaaa ggagtgaccc agaatgggag attgataaca    240
   gccaatccca tagttactga caaagaaaaa ccagtcaaca ttgagacaga accacctttt    300
   ggtgagagct acatcatagt aggggcaggt gaaaaagctt tgaaactaag ctggttcaag    360
   aaaggaagca gcatagggaa aatgttcgaa gcaaccgccg gaggatcagg agggaaagga    420
   atgtcatact ctatgtgtac aggaaagttt aaaattgtga aggaaatagc agaaacacaa    480
   catggaacaa tagttatcag agtacaatat gaaggggacg gctctccatg taagatccct    540
   tttgagataa tggatttgga aaaaagacac gtcttaggtc gcctgattac agttaacccg    600
   atcgtaacag aaaaagatag cccagtcaac atagaagcag aacctccatt cggagacagc    660
   tacatcatca taggagtaga gccgggacaa ttgaaactca actggtttaa gaaaggaagt    720
   tccatcggcc aaatgtttga gacaacaatg ggaggatcta aggggatgag ctatgcaatg    780
   tgcttgaata cctttgtgtt gaagaaagaa gtctccgaaa cgcagcatgg gacaatactc    840
   attaaggttg agtacaaagg ggaagatgca ccttgcaaga ttcctttctc cacagaggat    900
   ggacaaggga aagctcacaa tggtagactg atcacagcca acccagtggt gaccaagaag    960
   gaggagcctg tcaacattga ggctgaacct ccttttgggg aaagtaacat agtgattgga   1020
   attggagaca aagccttgaa aattaactgg tacaagaagg gaagctcgat tgggaagatg   1080
   ttcgaggcca ctgccggtgg ttctggtggt aagggaatgt catacacgat gtgctcagga   1140
   aagttctcaa ttgataaaga gatggcagaa acacagcatg ggacaacagt ggtgaaagtc   1200
   aagtatgagg gtgctggagc tccatgtaaa gttcccatag agataagaga tgtgaacaag   1260
   gaaaaagtgg ttgggcgtat catctcatct accccttttg ctgagaatac caacagtgtg   1320
   accaatatag aattggaacc cccttttggg gatagctaca tagtaatagg tgtaggagac   1380
   agtgcattaa cactccattg gttcaggaaa gggagctcca ttggcaagat gtttgagtcc   1440
   acatacagcg gccgcgtgga gacttgggct ttgagacacc catagtaagc gcgc         1494
<210> 18
   <211> 390
   <212> DNA
   <213> Flavivirus sp.
<400> 18
   cgtacgatgc tgctatccgt gccgttgctg ctcggcctcc tcggcctggc cgtcgccaga 60
   tctaagggaa caacctatgg cgtctgttca aaggctttca agtttcttgg gactcccgca 120
   gacacaggtc acggcactgt ggtgttggaa ttgcagtaca ctggcacgga tggaccttgc 180
   aaagttccta tctcgtcagt ggcttcattg aacgacctaa cgccagtggg cagattggtc 240
   actgtcaacc cttttgtttc agtggccacg gccaacgcta aggtcctgat tgaattggaa 300
   ccaccctttg gagactcata catagtggtg ggcagaggag aacaacagat taatcaccat 360
   tggcacaagt agcggccgct ataagcgcgc 390
<210> 19
   <211> 528
   <212> DNA
   <213> Flavivirus sp.
<400> 19
   cgtacgatgc tgctatccgt gccgttgctg ctcggcctcc tcggcctggc cgtcgccaga 60
   tctaagggaa caacctatgg cgtctgttca aaggctttca agtttcttgg gactcccgca 120
   gacacaggtc acggcactgt ggtgttggaa ttgcagtaca ctggcacgga tggaccttgc 180
   aaagttccta tctcgtcagt ggcttcattg aacgacctaa cgccagtggg cagattggtc 240
   actgtcaacc cttttgtttc agtggccacg gccaacgcta aggtcctgat tgaattggaa 300
   ccaccctttg gagactcata catagtggtg ggcagaggag aacaacagat taatcaccat 360
   tggcacaaga gacgcagtcg gaggtcactg acagtgcaga cacacggaga aagcactcta 420
   gcgaacaaga agggggcttg gatggacagc accaaggcca caaggtattt ggtaaaaaca 480
   gaatcatgga tcttgaggaa cccttgatag cggccgctat aagcgcgc 528
<210> 20
   <211> 624
   <212> PRT
   <213> Artificial
<220>
   <223> fusion sequence comprising domain(s) of Flaviviridae strains.
<400> 20
   Met Leu Leu Ser Val Pro Leu Leu Leu Gly Leu Leu Gly Leu Ala Val
   1               5                   10                  15
   Ala Asp Lys Leu Thr Leu Lys Gly Met Ser Tyr Val Met Cys Thr Gly
               20                  25                  30
   Ser Phe Lys Leu Glu Lys Glu Val Ala Glu Thr Gln His Gly Thr Val
           35                  40                  45
   Leu Val Gln Val Lys Tyr Glu Gly Thr Asp Ala Pro Cys Lys Ile Pro
       50                  55                  60
   Phe Ser Thr Gln Asp Glu Lys Gly Val Thr Gln Asn Gly Arg Leu Ile
   65                  70                  75                  80
   Thr Ala Asn Pro Ile Val Thr Asp Lys Glu Lys Pro Val Asn Ile Glu
                   85                  90                  95
   Thr Glu Pro Pro Phe Gly Glu Ser Tyr Ile Ile Val Gly Ala Gly Glu
               100                 105                 110
   Lys Ala Leu Lys Leu Ser Trp Phe Arg Arg Asp Lys Arg Ser Val Ala
           115                 120                 125
   Leu Ala Pro His Val Gly Leu Gly Leu Glu Thr Arg Thr Glu Thr Trp
       130                 135                 140
   Met Ser Ser Glu Gly Ala Trp Lys Gln Ile Gln Lys Val Glu Thr Trp
   145                 150                 155                 160
   Ala Leu Arg His Pro Arg Arg Asp Lys Arg Asp Lys Leu Gln Leu Lys
                   165                 170                 175
   Gly Met Ser Tyr Ser Met Cys Thr Gly Lys Phe Lys Ile Val Lys Glu
               180                 185                 190
   Ile Ala Glu Thr Gln His Gly Thr Ile Val Ile Arg Val Gln Tyr Glu
           195                 200                 205
   Gly Asp Gly Ser Pro Cys Lys Ile Pro Phe Glu Ile Thr Asp Leu Glu
       210                 215                 220
   Lys Arg His Val Leu Gly Arg Leu Ile Thr Val Asn Pro Ile Val Thr
   225                 230                 235                 240
   Glu Lys Asp Ser Pro Val Asn Ile Glu Ala Glu Pro Pro Phe Gly Asp
                   245                 250                 255
   Ser Tyr Ile Ile Val Gly Val Glu Pro Gly Gln Leu Lys Leu Asn Trp
   260 265 270
   Phe Arg Arg Asp Lys Arg Ser Val Ala Leu Ala Pro His Val Gly Leu
   275 280 285
   Gly Leu Glu Thr Arg Thr Glu Thr Trp Met Ser Ser Glu Gly Ala Trp
   290 295 300
   Lys Gln Ile Gln Lys Val Glu Thr Trp Ala Leu Arg His Pro Arg Arg
   305 310 315 320
   Asp Lys Arg Asp Lys Leu Lys Leu Lys Gly Met Ser Tyr Ala Met Cys
   325 330 335
   Leu Asn Thr Phe Val Leu Lys Lys Glu Val Ser Glu Thr Gln His Gly
   340 345 350
   Thr Ile Leu Ile Lys Val Glu Tyr Lys Gly Glu Asp Ala Pro Cys Lys
   355 360 365
   Ile Pro Phe Ser Thr Glu Asp Gly Gln Gly Lys Ala His Asn Gly Arg
   370 375 380
   Leu Ile Thr Ala Asn Pro Val Val Thr Lys Lys Glu Glu Pro Val Asn
   385 390 395 400
   Ile Glu Ala Glu Pro Pro Phe Gly Glu Ser Asn Ile Val Ile Gly Ile
   405 410 415
   Gly Asp Lys Ala Leu Lys Ile Asn Trp Tyr Arg Arg Asp Lys Arg Ser
   420 425 430
   Val Ala Leu Ala Pro His Val Gly Leu Gly Leu Glu Thr Arg Thr Glu
   435 440 445
   Thr Trp Met Ser Ser Glu Gly Ala Trp Lys Gln Ile Gln Lys Val Glu
   450 455 460
   Thr Trp Ala Leu Arg His Pro Arg Arg Asp Lys Arg Glu Lys Leu Arg
   465 470 475 480
   Ile Lys Gly Met Ser Tyr Thr Met Cys Ser Gly Lys Phe Ser Ile Asp
   485 490 495
   Lys Glu Met Ala Glu Thr Gln His Gly Thr Thr Val Val Lys Val Lys
   500 505 510
   Tyr Glu Gly Ala Gly Ala Pro Cys Lys Val Pro Ile Glu Ile Arg Asp
   515 520 525
   Val Asn Lys Glu Lys Val Val Gly Arg Ile Ile Ser Ser Thr Pro Leu
   530 535 540
   Ala Glu Asn Thr Asn Ser Val Thr Asn Ile Glu Leu Glu Pro Pro Phe
   545 550 555 560
   Gly Asp Ser Tyr Ile Val Ile Gly Val Gly Asn Ser Ala Leu Thr Leu
   565 570 575
   His Trp Phe Arg Arg Asp Lys Arg Ser Val Ala Leu Ala Pro His Val
   580 585 590
   Gly Leu Gly Leu Glu Thr Arg Thr Glu Thr Trp Met Ser Ser Glu Gly
   595 600 605
   Ala Trp Lys Gln Ile Gln Lys Val Glu Thr Trp Ala Leu Arg His Pro
   610 615 620
<210> 21
   <211> 609
   <212> PRT
   <213> Artificial
<220>
   <223> fusion sequence comprising domain(s) of Flaviviridae strains.
<400> 21
   Met Leu Leu Ser Val Pro Leu Leu Leu Gly Leu Leu Gly Leu Ala Val
   1 5 10 15
   Ala Asp Lys Leu Thr Leu Lys Gly Met Ser Tyr Val Met Cys Thr Gly
   20 25 30
   Ser Phe Lys Leu Glu Lys Glu Val Ala Glu Thr Gln His Gly Thr Val
   35 40 45
   Leu Val Gln Val Lys Tyr Glu Gly Thr Asp Ala Pro Cys Lys Ile Pro
   50 55 60
   Phe Ser Thr Gln Asp Glu Lys Gly Val Thr Gln Asn Gly Arg Leu Ile
   65 70 75 80
   Thr Ala Asn Pro Ile Val Thr Asp Lys Glu Lys Pro Val Asn Ile Glu
   85 90 95
   Thr Glu Pro Pro Phe Gly Glu Ser Tyr Ile Ile Val Gly Ala Gly Glu
   100 105 110
   Lys Ala Leu Lys Leu Ser Trp Phe Ser Val Ala Leu Ala Pro His Val
   115 120 125
   Gly Leu Gly Leu Glu Thr Arg Thr Glu Thr Trp Met Ser Ser Glu Gly
   130 135 140
   Ala Trp Lys Gln Ile Gln Lys Val Glu Thr Trp Ala Leu Arg His Pro
   145 150 155 160
   Arg Arg Asp Lys Arg Asp Lys Leu Gln Leu Lys Gly Met Ser Tyr Ser
   165 170 175
   Met Cys Thr Gly Lys Phe Lys Ile Val Lys Glu Ile Ala Glu Thr Gln
   180 185 190
   His Gly Thr Ile Val Ile Arg Val Gln Tyr Glu Gly Asp Gly Ser Pro
   195 200 205
   Cys Lys Ile Pro Phe Glu Ile Thr Asp Leu Glu Lys Arg His Val Leu
   210 215 220
   Gly Arg Leu Ile Thr Val Asn Pro Ile Val Thr Glu Lys Asp Ser Pro
   225 230 235 240
   Val Asn Ile Glu Ala Glu Pro Pro Phe Gly Asp Ser Tyr Ile Ile Val
   245 250 255
   Gly Val Glu Pro Gly Gln Leu Lys Leu Asn Trp Phe Ser Val Ala Leu
   260 265 270
   Ala Pro His Val Gly Leu Gly Leu Glu Thr Arg Thr Glu Thr Trp Met
   275 280 285
   Ser Ser Glu Gly Ala Trp Lys Gln Ile Gln Lys Val Glu Thr Trp Ala
   290 295 300
   Leu Arg His Pro Arg Arg Asp Lys Arg Asp Lys Leu Lys Leu Lys Gly
   305 310 315 320
   Met Ser Tyr Ala Met Cys Leu Asn Thr Phe Val Leu Lys Lys Glu Val
   325 330 335
   Ser Glu Thr Gln His Gly Thr Ile Leu Ile Lys Val Glu Tyr Lys Gly
   340 345 350
   Glu Asp Ala Pro Cys Lys Ile Pro Phe Ser Thr Glu Asp Gly Gln Gly
   355 360 365
   Lys Ala His Asn Gly Arg Leu Ile Thr Ala Asn Pro Val Val Thr Lys
   370 375 380
   Lys Glu Glu Pro Val Asn Ile Glu Ala Glu Pro Pro Phe Gly Glu Ser
   385 390 395 400
   Asn Ile Val Ile Gly Ile Gly Asp Lys Ala Leu Lys Ile Asn Trp Tyr
   405 410 415
   Ser Val Ala Leu Ala Pro His Val Gly Leu Gly Leu Glu Thr Arg Thr
   420 425 430
   Glu Thr Trp Met Ser Ser Glu Gly Ala Trp Lys Gln Ile Gln Lys Val
   435 440 445
   Glu Thr Trp Ala Leu Arg His Pro Arg Arg Asp Lys Arg Glu Lys Leu
   450 455 460
   Arg Ile Lys Gly Met Ser Tyr Thr Met Cys Ser Gly Lys Phe Ser Ile
   465 470 475 480
   Asp Lys Glu Met Ala Glu Thr Gln His Gly Thr Thr Val Val Lys Val
   485 490 495
   Lys Tyr Glu Gly Ala Gly Ala Pro Cys Lys Val Pro Ile Glu Ile Arg
   500 505 510
   Asp Val Asn Lys Glu Lys Val Val Gly Arg Ile Ile Ser Ser Thr Pro
   515 520 525
   Leu Ala Glu Asn Thr Asn Ser Val Thr Asn Ile Glu Leu Glu Pro Pro
   530 535 540
   Phe Gly Asp Ser Tyr Ile Val Ile Gly Val Gly Asn Ser Ala Leu Thr
   545 550 555 560
   Leu His Trp Phe Arg Arg Asp Lys Arg Ser Val Ala Leu Ala Pro His
   565 570 575
   Val Gly Leu Gly Leu Glu Thr Arg Thr Glu Thr Trp Met Ser Ser Glu
   580 585 590
   Gly Ala Trp Lys Gln Ile Gln Lys Val Glu Thr Trp Ala Leu Arg His
   595 600 605
   Pro
<210> 22
   <211> 624
   <212> PRT
   <213> Artificial
<220>
   <223> fusion sequence comprising domain(s) of Flaviviridae strains.
<400> 22
   Met Leu Leu Ser Val Pro Leu Leu Leu Gly Leu Leu Gly Leu Ala Val
   1 5 10 15
   Ala Asp Lys Leu Thr Leu Lys Gly Met Ser Tyr Val Met Cys Thr Gly
                   20                  25                  30
   Ser Phe Lys Leu Glu Lys Glu Val Ala Glu Thr Gln His Gly Thr Val
           35                  40                  45
   Leu Val Gln Val Lys Tyr Glu Gly Thr Asp Ala Pro Cys Lys Ile Pro
       50                  55                  60
   Phe Ser Thr Gln Asp Glu Lys Gly Val Thr Gln Asn Gly Arg Leu Ile
   65                  70                  75                  80
   Thr Ala Asn Pro Ile Val Thr Asp Lys Glu Lys Pro Val Asn Ile Glu
                   85                  90                  95
   Thr Glu Pro Pro Phe Gly Glu Ser Tyr Ile Ile Val Gly Ala Gly Glu
               100                 105                 110
   Lys Ala Leu Lys Leu Ser Trp Phe Arg Arg Asp Lys Arg Ser Val Ala
           115                 120                 125
   Leu Ala Pro His Val Gly Leu Gly Leu Glu Thr Arg Thr Glu Thr Trp
       130                 135                 140
   Met Ser Ser Glu Gly Ala Trp Lys Gln Ile Gln Lys Val Glu Thr Trp
   145                 150                 155                 160
   Ala Leu Arg His Pro Arg Arg Asp Lys Arg Asp Lys Leu Gln Leu Lys
                   165                 170                 175
   Gly Met Ser Tyr Ser Met Cys Thr Gly Lys Phe Lys Ile Val Lys Glu
               180                 185                 190
   Ile Ala Glu Thr Gln His Gly Thr Ile Val Ile Arg Val Gln Tyr Glu
           195                 200                 205
   Gly Asp Gly Ser Pro Cys Lys Ile Pro Phe Glu Ile Thr Asp Leu Glu
       210                 215                 220
   Lys Arg His Val Leu Gly Arg Leu Ile Thr Val Asn Pro Ile Val Thr
   225                 230                 235                 240
   Glu Lys Asp Ser Pro Val Asn Ile Glu Ala Glu Pro Pro Phe Gly Asp
                   245                 250                 255
   Ser Tyr Ile Ile Val Gly Val Glu Pro Gly Gln Leu Lys Leu Asn Trp
               260                 265                 270
   Phe Arg Arg Glu Lys Arg Ser Val Ala Leu Val Pro His Val Gly Met
           275                 280                 285
   Gly Leu Glu Thr Arg Thr Glu Thr Trp Met Ser Ser Glu Gly Ala Trp
   290 295 300
   Lys His Val Gln Arg Ile Glu Thr Trp Ile Leu Arg His Pro Arg Arg
   305 310 315 320
   Asp Lys Arg Asp Lys Leu Lys Leu Lys Gly Met Ser Tyr Ala Met Cys
   325 330 335
   Leu Asn Thr Phe Val Leu Lys Lys Glu Val Ser Glu Thr Gln His Gly
   340 345 350
   Thr Ile Leu Ile Lys Val Glu Tyr Lys Gly Glu Asp Ala Pro Cys Lys
   355 360 365
   Ile Pro Phe Ser Thr Glu Asp Gly Gln Gly Lys Ala His Asn Gly Arg
   370 375 380
   Leu Ile Thr Ala Asn Pro Val Val Thr Lys Lys Glu Glu Pro Val Asn
   385 390 395 400
   Ile Glu Ala Glu Pro Pro Phe Gly Glu Ser Asn Ile Val Ile Gly Ile
   405 410 415
   Gly Asp Lys Ala Leu Lys Ile Asn Trp Tyr Arg Arg Asp Lys Arg Ser
   420 425 430
   Val Ala Leu Ala Pro His Val Gly Met Gly Leu Asp Thr Arg Thr Gln
   435 440 445
   Thr Trp Met Ser Ala Glu Gly Ala Trp Arg Gln Val Glu Lys Val Glu
   450 455 460
   Thr Trp Ala Leu Arg His Pro Arg Arg Asp Lys Arg Glu Lys Leu Arg
   465 470 475 480
   Ile Lys Gly Met Ser Tyr Thr Met Cys Ser Gly Lys Phe Ser Ile Asp
   485 490 495
   Lys Glu Met Ala Glu Thr Gln His Gly Thr Thr Val Val Lys Val Lys
   500 505 510
   Tyr Glu Gly Ala Gly Ala Pro Cys Lys Val Pro Ile Glu Ile Arg Asp
   515 520 525
   Val Asn Lys Glu Lys Val Val Gly Arg Ile Ile Ser Ser Thr Pro Leu
   530 535 540
   Ala Glu Asn Thr Asn Ser Val Thr Asn Ile Glu Leu Glu Pro Pro Phe
   545 550 555 560
   Gly Asp Ser Tyr Ile Val Ile Gly Val Gly Asn Ser Ala Leu Thr Leu
   565 570 575
   His Trp Phe Arg Arg Glu Lys Arg Ser Val Ala Leu Thr Pro His Ser
   580 585 590
   Gly Met Gly Leu Glu Thr Arg Ala Glu Thr Trp Met Ser Ser Glu Gly
   595 600 605
   Ala Trp Lys His Ala Gln Arg Val Glu Ser Trp Ile Leu Arg Asn Pro
   610 615 620
<210> 23
   <211> 489
   <212> PRT
   <213> Artificial
<220>
   <223> fusion sequence comprising domaine) of Flaviviridae strains.
<400> 23
   Met Leu Leu Ser Val Pro Leu Leu Leu Gly Leu Leu Gly Leu Ala Val
   1 5 10 15
   Ala Asp Lys Leu Thr Leu Lys Gly Met Ser Tyr Val Met Cys Thr Gly
   20 25 30
   Ser Phe Lys Leu Glu Lys Glu Val Ala Glu Thr Gln His Gly Thr Val
   35 40 45
   Leu Val Gln Val Lys Tyr Glu Gly Thr Asp Ala Pro Cys Lys Ile Pro
   50 55 60
   Phe Ser Thr Gln Asp Glu Lys Gly Val Thr Gln Asn Gly Arg Leu Ile
   65 70 75 80
   Thr Ala Asn Pro Ile Val Thr Asp Lys Glu Lys Pro Val Asn Ile Glu
   85 90 95
   Thr Glu Pro Pro Phe Gly Glu Ser Tyr Ile Ile Val Gly Ala Gly Glu
   100 105 110
   Lys Ala Leu Lys Leu Ser Trp Phe Arg Arg Asp Lys Arg Asp Lys Leu
   115 120 125
   Gln Leu Lys Gly Met Ser Tyr Ser Met Cys Thr Gly Lys Phe Lys Ile
   130 135 140
   Val Lys Glu Ile Ala Glu Thr Gln His Gly Thr Ile Val Ile Arg Val
   145 150 155 160
   Gln Tyr Glu Gly Asp Gly Ser Pro Cys Lys Ile Pro Phe Glu Ile Thr
   165 170 175
   Asp Leu Glu Lys Arg His Val Leu Gly Arg Leu Ile Thr Val Asn Pro
   180 185 190
   Ile Val Thr Glu Lys Asp Ser Pro Val Asn Ile Glu Ala Glu Pro Pro
   195 200 205
   Phe Gly Asp Ser Tyr Ile Ile Val Gly Val Glu Pro Gly Gln Leu Lys
   210 215 220
   Leu Asn Trp Phe Arg Arg Asp Lys Arg Asp Lys Leu Lys Leu Lys Gly
   225 230 235 240
   Met Ser Tyr Ala Met Cys Leu Asn Thr Phe Val Leu Lys Lys Glu Val
   245 250 255
   Ser Glu Thr Gln His Gly Thr Ile Leu Ile Lys Val Glu Tyr Lys Gly
   260 265 270
   Glu Asp Ala Pro Cys Lys Ile Pro Phe Ser Thr Glu Asp Gly Gln Gly
   275 280 285
   Lys Ala His Asn Gly Arg Leu Ile Thr Ala Asn Pro Val Val Thr Lys
   290 295 300
   Lys Glu Glu Pro Val Asn Ile Glu Ala Glu Pro Pro Phe Gly Glu Ser
   305 310 315 320
   Asn Ile Val Ile Gly Ile Gly Asp Lys Ala Leu Lys Ile Asn Trp Tyr
   325 330 335
   Arg Arg Asp Lys Arg Glu Lys Leu Arg Ile Lys Gly Met Ser Tyr Thr
   340 345 350
   Met Cys Ser Gly Lys Phe Ser Ile Asp Lys Glu Met Ala Glu Thr Gln
   355 360 365
   His Gly Thr Thr Val Val Lys Val Lys Tyr Glu Gly Ala Gly Ala Pro
   370 375 380
   Cys Lys Val Pro Ile Glu Ile Arg Asp Val Asn Lys Glu Lys Val Val
   385 390 395 400
   Gly Arg Ile Ile Ser Ser Thr Pro Leu Ala Glu Asn Thr Asn Ser Val
   405 410 415
   Thr Asn Ile Glu Leu Glu Pro Pro Phe Gly Asp Ser Tyr Ile Val Ile
   420 425 430
   Gly Val Gly Asn Ser Ala Leu Thr Leu His Trp Phe Arg Arg Asp Lys
   435 440 445
   Arg Ser Val Ala Leu Ala Pro His Val Gly Leu Gly Leu Glu Thr Arg
   450 455 460
   Thr Glu Thr Trp Met Ser Ser Glu Gly Ala Trp Lys Gln Ile Gln Lys
   465 470 475 480
   Val Glu Thr Trp Ala Leu Arg His Pro
   485
<210> 24
   <211> 474
   <212> PRT
   <213> Artificial
<220>
   <223> fusion sequence comprising domain(s) of Flaviviridae strains.
<400> 24
   Met Leu Leu Ser Val Pro Leu Leu Leu Gly Leu Leu Gly Leu Ala Val
   1 5 10 15
   Ala Asp Lys Leu Thr Leu Lys Gly Met Ser Tyr Val Met Cys Thr Gly
   20 25 30
   Ser Phe Lys Leu Glu Lys Glu Val Ala Glu Thr Gln His Gly Thr Val
   35 40 45
   Leu Val Gln Val Lys Tyr Glu Gly Thr Asp Ala Pro Cys Lys Ile Pro
   50 55 60
   Phe Ser Thr Gln Asp Glu Lys Gly Val Thr Gln Asn Gly Arg Leu Ile
   65 70 75 80
   Thr Ala Asn Pro Ile Val Thr Asp Lys Glu Lys Pro Val Asn Ile Glu
   85 90 95
   Thr Glu Pro Pro Phe Gly Glu Ser Tyr Ile Ile Val Gly Ala Gly Glu
   100 105 110
   Lys Ala Leu Lys Leu Ser Trp Phe Asp Lys Leu Gln Leu Lys Gly Met
   115 120 125
   Ser Tyr Ser Met Cys Thr Gly Lys Phe Lys Ile Val Lys Glu Ile Ala
   130 135 140
   Glu Thr Gln His Gly Thr Ile Val Ile Arg Val Gln Tyr Glu Gly Asp
   145 150 155 160
   Gly Ser Pro Cys Lys Ile Pro Phe Glu Ile Thr Asp Leu Glu Lys Arg
   165 170 175
   His Val Leu Gly Arg Leu Ile Thr Val Asn Pro Ile Val Thr Glu Lys
   180 185 190
   Asp Ser Pro Val Asn Ile Glu Ala Glu Pro Pro Phe Gly Asp Ser Tyr
   195 200 205
   Ile Ile Val Gly Val Glu Pro Gly Gln Leu Lys Leu Asn Trp Phe Asp
   210 215 220
   Lys Leu Lys Leu Lys Gly Met Ser Tyr Ala Met Cys Leu Asn Thr Phe
   225 230 235 240
   Val Leu Lys Lys Glu Val Ser Glu Thr Gln His Gly Thr Ile Leu Ile
   245 250 255
   Lys Val Glu Tyr Lys Gly Glu Asp Ala Pro Cys Lys Ile Pro Phe Ser
   260 265 270
   Thr Glu Asp Gly Gln Gly Lys Ala His Asn Gly Arg Leu Ile Thr Ala
   275 280 285
   Asn Pro Val Val Thr Lys Lys Glu Glu Pro Val Asn Ile Glu Ala Glu
   290 295 300
   Pro Pro Phe Gly Glu Ser Asn Ile Val Ile Gly Ile Gly Asp Lys Ala
   305 310 315 320
   Leu Lys Ile Asn Trp Tyr Glu Lys Leu Arg Ile Lys Gly Met Ser Tyr
   325 330 335
   Thr Met Cys Ser Gly Lys Phe Ser Ile Asp Lys Glu Met Ala Glu Thr
   340 345 350
   Gln His Gly Thr Thr Val Val Lys Val Lys Tyr Glu Gly Ala Gly Ala
   355 360 365
   Pro Cys Lys Val Pro Ile Glu Ile Arg Asp Val Asn Lys Glu Lys Val
   370 375 380
   Val Gly Arg Ile Ile Ser Ser Thr Pro Leu Ala Glu Asn Thr Asn Ser
   385 390 395 400
   Val Thr Asn Ile Glu Leu Glu Pro Pro Phe Gly Asp Ser Tyr Ile Val
   405 410 415
   Ile Gly Val Gly Asn Ser Ala Leu Thr Leu His Trp Phe Arg Arg Asp
   420 425 430
   Lys Arg Ser Val Ala Leu Ala Pro His Val Gly Leu Gly Leu Glu Thr
   435 440 445
   Arg Thr Glu Thr Trp Met Ser Ser Glu Gly Ala Trp Lys Gln Ile Gln
   450 455 460
   Lys Val Glu Thr Trp Ala Leu Arg His Pro
   465 470
<210> 25
   <211> 1890
   <212> DNA
   <213> Artificial
<220>
   <223> fusion sequence comprising domain(s) of Flaviviridae strains.
<400> 25
   cgtacgatgc tcctttccgt cccgttgcta ttaggactac tcgggttggc tgtcgccgat     60
   aagctgactc tgaagggaat gtcatacgtt atgtgcacag ggagcttcaa gttggagaag    120
   gaggtggcag agacccagca cggaacagtc ttggtgcagg tcaagtacga gggcacggac    180
   gctccttgta agatcccatt ctcaacccag gatgagaagg gcgtgaccca gaatggacgg    240
   ctgatcacgg ctaatcccat cgtgactgat aaggagaagc cagtcaacat cgagacagag    300
   ccacccttcg gtgagtctta cataatagtc ggggccggag agaaggctct caagctgagt    360
   tggttcaggc gggataagcg aagcgtcgca cttgccccac acgttggcct cggactcgag    420
   acaagaacag agacctggat gagcagcgag ggagcctgga agcagattca gaaggttgag    480
   acctgggctc tccggcaccc tcgccgtgat aagagagaca agctgcagct taaggggatg    540
   agttattcca tgtgcacagg caagttcaag attgtcaagg agatagcaga gactcagcac    600
   ggaaccatag tgatcagagt tcagtacgag ggagacggat ccccatgcaa gattccgttt    660
   gagattaccg acctggagaa gcgccacgtg ctggggagac tcattactgt gaacccaatc    720
   gtgactgaga aggattctcc cgtcaatatc gaggctgagc caccattcgg agattcttat    780
   ataatcgttg gtgtagagcc tggccagttg aagttgaatt ggtttaggcg ggataagagg    840
   agcgtggctc tcgctccaca tgttggcctg ggcctggaga cccgaacaga gacgtggatg    900
   agctccgaag gtgcctggaa gcagattcag aaggttgaga cctgggccct gcgacaccct    960
   agacgggata agcgcgataa gctgaagctt aagggtatgt cctacgcaat gtgcctgaac   1020
   acgttcgtgc tgaagaagga ggtttcagag acccagcacg ggacaattct cattaaggtg   1080
   gagtacaagg gcgaggacgc gccctgcaag atcccgttca gtactgaaga tggacagggc   1140
   aaggctcaca atgggcgact cattactgct aatccagtgg tgaccaagaa ggaagagcca   1200
   gtgaatatag aggcagagcc accatttgga gagagcaaca tcgtgatagg tatcggcgat   1260
   aaggcactga agatcaactg gtatcgccgc gacaagcgat ccgtggcatt ggcgcctcat   1320
   gtgggcctgg gtctagagac ccgcacagag acgtggatga gtagcgaagg cgcgtggaag   1380
   cagatccaga aggtcgagac ttgggcactg cggcaccctc gacgtgacaa gcgagagaag   1440
   ctccgaatca agggaatgag ttacacaatg tgcagcggca agttctcaat tgataaggag   1500
   atggccgaga cccagcacgg cacaaccgtg gtcaaggtca agtacgaagg tgcgggcgct   1560
   ccttgcaagg tcccaatcga gattagggat gtgaacaagg agaaggtcgt gggaagaatc   1620
   attagttcca cgcctcttgc tgagaacact aatagtgtca ccaatatcga gctagagcca   1680
   cccttcggag attcttacat tgtcattgga gttggcaatt ccgctcttac tctgcattgg   1740
   tttaggagag ataagagaag cgtggccctg gcaccacacg tgggattagg gctggagaca   1800
   cgaaccgaga cctggatgag cagcgaagga gcctggaagc agattcagaa ggtcgagact   1860
   tgggccctca gacacccttg atgagcgcgc                                      1890
<210> 26
   <211> 1842
   <212> DNA
   <213> Artificial
<220>
   <223> fusion sequence comprising domain(s) of Flaviviridae strains.
<400> 26
   cgtacgatgc tgttgagcgt gccattattg ctggggctgc tgggtctggc tgtggccgac      60
   aagcttaccc tgaagggtat gagctacgtg atgtgcactg gttcgttcaa gctagagaag     120
   gaggtggcag agacccagca tggaactgtg ttagtacaag ttaagtatga ggggaccgac     180
   gcaccctgta agattccatt ctctacccaa gacgagaagg gcgtcacgca gaatggtcgc     240
   ttgattacgg ctaatccaat agtgacggac aaggagaagc cagtgaatat cgagacagaa     300
   cctccatttg gagaatccta cataatagtc ggggccggcg agaaggccct gaagttgtcc     360
   tggttcagcg tggccctggc tccacacgtg ggcctgggcc tggagacccg aactgagact     420
   tggatgtcgt ccgagggcgc ctggaagcag atccagaagg tagagacttg ggccctgcgc     480
   catcctagga gagataagag ggacaagttg cagctcaagg gcatgtccta ctccatgtgt     540
   acaggtaagt tcaagattgt gaaggagatt gctgagaccc agcacggcac catcgtaata     600
   cgggtgcagt acgagggcga tggcagtcca tgtaagatac ctttcgagat cactgacctt     660
   gagaagcgcc acgtgcttgg gcggctcatt accgtcaatc caatcgtgac tgagaaggat     720
   agtccggtga atattgaggc tgagccgccg tttggggata gttatataat cgttggcgtg     780
   gagcccggac aactcaagtt gaattggttc tctgtggcct tggcaccaca cgtggggctg     840
   ggcctggaga cacggaccga gacttggatg tcaagtgaag gagcctggaa gcagattcag     900
   aaggtggaga cttgggccct tcggcaccct agacgtgata agcgcgacaa gctgaagctc     960
   aagggaatgt cttacgccat gtgtctgaat actttcgtcc tgaagaagga ggtgtccgag    1020
   actcagcatg ggactatcct gatcaaggtg gagtacaagg gtgaagacgc accatgtaag    1080
   ataccattca gcacagagga cggtcagggc aaggctcata acgggcgcct tataactgcc    1140
   aatccggttg tgaccaagaa ggaggagccc gtgaacatcg aggcagagcc accattcggc    1200
   gagtccaaca tcgtgatagg aatcggtgat aaggccctca agattaattg gtattcagtg    1260
   gccctggccc ctcacgtcgg actgggactc gagaccagga cagagacatg gatgtcttct    1320
   gagggtgcat ggaagcagat acagaaggtt gagacctggg ccctcaggca cccacgtagg    1380
   gacaagcggg agaagttgag gattaagggg atgtcgtaca ccatgtgcag cggcaagttc    1440
   tccattgaca aggagatggc agagacgcag cacggaacaa cggtcgttaa ggtcaagtac    1500
   gaaggagcgg gcgccccatg caaggtccca atcgagatta gggacgtgaa caaggagaag    1560
   gttgtaggac ggattattag tagcacccca ctggcggaga atacgaatag tgtcacgaat    1620
   atcgaactgg aaccaccttt cggcgatagc tacatcgtga ttggtgttgg caattccgca    1680
   cttacactgc actggtttcg gagagacaag cgaagcgtgg ccctggcccc tcacgtggga    1740
   cttggcctgg agacccgcac agagacttgg atgtcctctg aaggagcatg gaagcagatt    1800
   cagaaggtgg agacttgggc attacgccac ccatgagcgc gc                       1842
<210> 27
   <211> 1890
   <212> DNA
   <213> Artificial
<220>
   <223> fusion séquence comprising domain(s) of Flaviviridae strains.
<400> 27
   cgtacgatgc tgctatctgt gccactgctc ctgggactgc tggggctggc cgtggctgac     60
   aagttgacgc ttaaggggat gtcctacgta atgtgcacag gatcattcaa gctagagaag    120
   gaagttgcag agacgcagca cggcaccgtg ctggttcagg tgaagtacga gggcactgat    180
   gcaccctgca agatcccatt cagtactcag gacgagaagg gagtcacgca gaatgggagg    240
   ctcatcacgg ccaacccaat tgttaccgac aaggagaagc ccgttaatat agagactgaa    300
   ccgcccttcg gggagtcata cattattgtg ggcgcagggg agaaggctct gaagctttcc    360
   tggttccgta gggataagcg cagcgtggcg ctagcccctc atgtgggtct cgggctggag    420
   actcgcaccg agacatggat gtcatccgag ggtgcgtgga agcagatcca gaaggttgag    480
   acatgggcac ttcgccaccc acggcgcgac aagcgtgaca agcttcagct gaaggggatg    540
   tcttatagca tgtgcacagg caagttcaag atcgtcaagg agatagcaga gacacagcat    600
   ggcacaatag tcatacgggt gcaatatgag ggagatggct ctccgtgtaa gatacctttc    660
   gagatcaccg atctggagaa gcggcacgtc ctcgggagac taattacggt gaacccaatt    720
   gtgacagaga aggatagccc agttaatatt gaggccgaac cacccttcgg agacagctac    780
   attatcgtag gagtcgagcc agggcagctg aagctgaact ggttccgcag ggagaagcgt    840
   tccgtcgctc ttgttccaca tgtgggaatg ggcctggaga cccgcaccga gacatggatg    900
   agcagcgagg gtgcctggaa gcacgtacag agaattgaga cttggatctt aaggcatcct    960
   cggcgggata agcgcgacaa gttgaagctg aagggaatgt cttacgcaat gtgtttgaac   1020
   actttcgtcc tgaagaagga ggtgtcagag acccagcacg gaactatact tattaaggtt   1080
   gagtacaagg gagaagacgc tccttgcaag atcccattct caactgaaga tgggcagggc   1140
   aaggcacaca acggaaggct catcacagct aacccagtgg ttaccaagaa ggaggagccc   1200
   gtgaacatcg aagccgagcc gcccttcgga gagagcaaca tagtgatcgg tattggggat   1260
   aaggctctga agatcaattg gtatcgccgg gacaagagaa gtgtggccct ggccccacat 1320
   gtcggcatgg gcttggacac acggacacag acatggatgt ccgctgaagg agcctggagg 1380
   caggtggaga aggtcgagac ctgggcgctg cgccacccgc gtcgagacaa gagagagaag 1440
   ttaagaatca agggtatgtc ctacacgatg tgttccggta agttctccat agacaaggag 1500
   atggctgaga cccagcacgg cactacggtg gtcaaggtga agtatgaggg agccggcgca 1560
   ccttgcaagg tgccgatcga gatccgcgac gtcaacaagg agaaggtggt cgggcgcatc 1620
   atttccagta ccccattagc tgagaatact aatagcgtga ctaatattga gttggaacca 1680
   cccttcggag actcttacat cgttatcggg gtgggcaact cagctcttac acttcactgg 1740
   ttccgcaggg agaagagatc agtggcccta acccctcact caggcatggg cctggagact 1800
   cgggccgaga cctggatgag cagcgagggt gcctggaagc acgcccagcg agtggaatcg 1860
   tggattctta ggaatccatg atgagcgcgc 1890
<210> 28
   <211> 1482
   <212> DNA
   <213> Artificial
<220>
   <223> fusion sequence comprising domain(s) of Flaviviridae strains.
<400> 28
   cgtacgatgc tgttatctgt acctctcctg ctcggcctcc ttggactggc agttgcagat 60
   aagcttacac tcaagggaat gtcctatgtg atgtgtaccg ggtccttcaa gttggagaag 120
   gaggtagctg agacgcagca tggcacagtg ctggtgcagg ttaagtatga aggaactgat 180
   gctccgtgta agatcccgtt cagtactcag gatgagaagg gtgtgaccca gaatggccgc 240
   ttaattacag ccaaccctat cgtaactgac aaggagaagc ctgtgaacat cgagactgaa 300
   ccacccttcg gtgagagtta tataatagtt ggcgccggag agaaggccct caagcttagc 360
   tggtttcggc gcgacaagcg agataagctc caacttaagg gaatgtcata ctcaatgtgc 420
   acaggtaagt tcaagatagt gaaggagata gccgagaccc agcatggcac catcgtgatc 480
   agagtccaat atgaaggaga cgggtctcct tgtaagatcc cattcgagat aacagacttg 540
   gagaagaggc acgtgctggg acgtctgata acagttaatc ccatagttac cgagaaggat 600
   agccctgtta acattgaggc tgaacctccc ttcggagatt cctacattat tgtcggcgtg 660
   gagccaggtc agctgaagct taactggttc agacgggaca agcgcgacaa gctgaagttg 720
   aagggcatgt cctatgcaat gtgtctcaat actttcgttc tcaagaagga agtgagcgag 780
   acacagcatg gcaccatatt aattaaggtt gagtacaagg gtgaggatgc tccttgcaag 840
   attcctttca gcaccgagga tggacagggc aaggcccaca atggtcggct gatcaccgcc 900
   aaccctgtgg taaccaagaa ggaagagccc gtcaacatag aagccgagcc gccgtttggc 960
   gagtccaata tagtaatcgg catcggagat aaggccttga agattaattg gtacaggcgg 1020
   gataagcgcg agaagttacg gattaagggt atgtcttaca ctatgtgtag cggtaagttt 1080
   agcatcgaca aggagatggc agagacccag catggcacta ccgtggtcaa ggtgaagtac 1140
   gaaggagctg gagcaccatg taaggtccct atcgagatcc gcgacgtgaa caaggagaag 1200
   gtggtgggaa gaatcatctc ttccacacct ctggcagaga acacgaatag cgtgactaat 1260
   atcgaacttg agcctccttt cggcgattcc tacatcgtta ttggcgtggg caactccgcc 1320
   ctgacactgc attggtttag acgtgacaag cgtagcgtgg cccttgcacc acacgtgggc 1380
   ctaggcctgg agacgcgtac agagacatgg atgagcagtg aaggagcctg gaagcagatt 1440
   cagaaggtcg agacctgggc tctcagacat ccttgagcgc gc 1482
<210> 29
   <211> 1440
   <212> DNA
   <213> Artificial
<220>
   <223> fusion sequence comprising domain(s) of Flaviviridae strains.
<400> 29
   cgtacgatgc tcctaagcgt tccactgctt ctgggactgc tgggtttggc cgttgccgac 60
   aagctcacgc tcaaggggat gagctacgtc atgtgcacag gctcattcaa gttggagaag 120
   gaagtcgccg agacgcagca cggaacagta ctcgtgcagg tcaagtatga gggcaccgac 180
   gctccatgca agatcccgtt ctccacacag gacgagaagg gcgttactca gaacggaaga 240
   ttaattactg ccaatcctat agtaacagac aaggagaagc ctgtgaatat tgagactgag 300
   cctcccttcg gtgaatccta catcattgta ggggctggcg agaaggcttt gaagctctcc 360
   tggtttgata agctgcagct caagggaatg tcgtattcaa tgtgcactgg gaagttcaag 420
   attgtgaagg agattgcaga gactcaacac ggcaccattg tgataagggt ccagtacgag 480
   ggtgatgggt caccctgcaa gattcccttt gagatcacag accttgagaa gaggcacgta 540
   cttgggcgat taattaccgt gaatcctatt gtgactgaga aggatagtcc cgtgaacatt 600
   gaagctgagc ctcctttcgg agatagctac attatcgtgg gagtcgagcc tggccagttg 660
   aagctcaact ggtttgataa gctgaagctc aagggcatgt cctacgctat gtgcttgaat 720
   acatttgtgc tgaagaagga ggtgagtgag acccaacacg gaaccatcct gatcaaggtg 780
   gagtacaagg gtgaagatgc accttgcaag atccctttct ccactgaaga cgggcagggc 840
   aaggcccata atgggagact cataacagct aatccagtgg tcaccaagaa ggaagaacca 900
   gtcaacatcg aggcggagcc accatttggg gagagtaaca tcgtgatcgg tattggcgat 960
   aaggccctga agattaactg gtatgagaag ttgagaatta aggggatgag ctataccatg 1020
   tgctcaggta agttcagcat cgataaggag atggccgaga cacagcacgg gaccacagtt 1080
   gtgaaggtga agtacgaggg cgctggggcc ccatgcaagg tgcctatcga gattcgcgac 1140
   gtgaacaagg agaaggtcgt ggggcgaatc atctcatcca cccctctcgc agagaacacg 1200
   aactctgtga ccaatattga gctggaacca cctttcggag actcatatat cgtcataggc 1260
   gtcggcaatt cagctttgac attgcactgg ttcagacggg acaagcggtc cgttgccctg 1320
   gctccacacg tcggcctggg ccttgagact aggacggaga cctggatgtc ctccgagggc 1380
   gcctggaagc agatccagaa ggtggagacc tgggccctgc gccacccctg ataggcgcgc 1440
<210> 30
   <211> 40
   <212> PRT
   <213> Flavivirus sp.
<400> 30
   Ser Val Ala Leu Ala Pro His Val Gly Leu Gly Leu Glu Thr Arg Thr
   1               5                   10                  15
   Glu Thr Trp Met Ser Ser Glu Gly Ala Trp Lys Gln Ile Gln Lys Val
               20                  25                  30
   Glu Thr Trp Ala Leu Arg His Pro
           35                  40
<210> 31
   <211> 40
   <212> PRT
   <213> Flavivirus sp.
<400> 31
   Ser Val Ala Leu Ala Pro His Val Gly Met Gly Leu Asp Thr Arg Thr
   1               5                   10                  15
   Gln Thr Trp Met Ser Ala Glu Gly Ala Trp Arg Gln Val Glu Lys Val
               20                  25                  30
   Glu Thr Trp Ala Leu Arg His Pro
           35                  40
<210> 32
   <211> 40
   <212> PRT
   <213> Flavivirus sp.
<400> 32
   Ser Val Ala Leu Thr Pro His Ser Gly Met Gly Leu Glu Thr Arg Ala
   1               5                   10                  15
   Glu Thr Trp Met Ser Ser Glu Gly Ala Trp Lys His Ala Gln Arg Val
               20                  25                  30
   Glu Ser Trp Ile Leu Arg Asn Pro
           35                  40
<210> 33
   <211> 40
   <212> PRT
   <213> Flavivirus sp.
<400> 33
   Ser Val Ala Leu Val Pro His Val Gly Met Gly Leu Glu Thr Arg Thr
   1               5                   10                  15
   Glu Thr Trp Met Ser Ser Glu Gly Ala Trp Lys His Ala Gln Arg Ile
               20                  25                  30
   Glu Thr Trp Ile Leu Arg His Pro
           35                  40